# EUROPEAN PATENT SPECIFICATION

(11) **EP 2 625 196 B1**
(45) Date of publication and mention of the grant of the patent: **23.03.2016**
(21) Application number: 10784597.6
(22) Date of filing: 05.10.2010
(51) Int. Cl.: C07K 14/705, C12N 15/62, A61K 38/00

(54) **FUSION POLYPEPTIDES COMPRISING TIR AND DIMERIZATION DOMAIN FOR MODULATION OF TLR/INNATE IMMUNITY SIGNALING**
FUSIONSPOLYPEPTIDE MIT TIR UND DIMERISIERUNGSDOMÄNE ZUR SIGNALMODULATION VON TLR-INDUZIERTER/ANGEBORENER IMMUNITÄT
POLYPEPTIDES DE FUSION COMPRENANT TIR ET DOMAINE DE DIMÉRISATION POUR LA MODULATION DU SIGNAL D'IMMUNITÉ DE TLR/INNÉ

(43) Date of publication of application: 14.08.2013
(73) Proprietor: Kemijski Institut, 1000 Ljubljana (SI)
(72) Inventor: JERALA, Roman, 1000 Ljubljana (SI); FEKONJA, Ota, 1000 Ljubljana (SI)
(74) Representative: Grünecker Patent- und Rechtsanwälte PartG mbB
(86) International application number: PCT/SI2010/000055
(87) International publication number: WO 2012/047175

(56) References cited:
- WO-A1-2008/097875
- WO-A1-2010/050902
- WO-A2-2004/073641
- WO-A2-2006/113530
- WO-A2-2007/146959
- WO-A8-2006/035066
- JIN MI SUN ET AL: "Structures of the toll-like receptor family and its ligand complexes", IMMUNITY, vol. 29, no. 2, August 2008 (2008-08), pages 182-191, XP002633061, ISSN: 1074-7613
- O'NEILL ET AL: "How Toll-like receptors signal: what we know and what we don't know", CURRENT OPINION IN IMMUNOLOGY, ELSEVIER, OXFORD, GB, vol. 18, no. 1, 1 February 2006 (2006-02-01), pages 3-9, XP025078962, ISSN: 0952-7915, DOI: DOI:10.1016/J.COI.2005.11.012 [retrieved on 2006-02-01]
- MARTIN M U ET AL: "Summary and comparison of the signaling mechanisms of the Toll/interleukin-1 receptor family", BIOCHIMICA ET BIOPHYSICA ACTA, ELSEVIER, NL, vol. 1592, no. 3, 11 November 2002 (2002-11-11), pages 265-280, XP002323152, ISSN: 0006-3002, DOI: DOI:10.1016/S0167-4889(02)00320-8
- GUAN YUE ET AL: "Human TLRs 10 and 1 Share Common Mechanisms of Innate Immune Sensing but Not Signaling", JOURNAL OF IMMUNOLOGY, vol. 184, no. 9, May 2010 (2010-05), pages 5094-5103, XP002633062, ISSN: 0022-1767
- THAKRAN SHALINI ET AL: "Identification of Francisella tularensis lipoproteins that stimulate the toll-like receptor (TLR)2/TLR1 heterodimer", JOURNAL OF BIOLOGICAL CHEMISTRY, vol. 283, no. 7, February 2008 (2008-02), pages 3751-3760, XP002633063, ISSN: 0021-9258
- ZHANG H ET AL: "Integrin-nucleated Toll-like receptor (TLR) dimerization reveals subcellular targeting of TLRs and distinct mechanisms of TLR4 activation and signaling", FEBS LETTERS, ELSEVIER, AMSTERDAM, NL, vol. 532, no. 1-2, 4 December 2002 (2002-12-04), pages 171-176, XP004395364, ISSN: 0014-5793, DOI: DOI:10.1016/S0014-5793(02)03669-4
- FORD K G ET AL: "PROTEIN TRANSDUCTION: AN ALTERNATIVE TO GENETIC INTERVENTION?", GENE THERAPY, MACMILLAN PRESS LTD., BASINGSTOKE, GB, vol. 8, no. 1, 1 January 2001 (2001-01-01) , pages 1-04, XP001098109, ISSN: 0969-7128, DOI: DOI:10.1038/SJ.GT.3301383
- XU YINGWU ET AL: "Structural basis for signal transduction by the Toll/interleukin-1 receptor domains", NATURE, NATURE PUBLISHING GROUP, LONDON, GB, vol. 408, no. 6808, 1 January 2000 (2000-01-01), pages 111-115, XP002183508, ISSN: 0028-0836, DOI: DOI:10.1038/35047056

## Description

### Field of invention

The field of the invention is the fusion polypeptide comprising TIR and dimerization domain wherein the two are connected by linker peptide and the said fusion polypeptide has improved inhibition of TLR/IL-1R signaling. The field of the invention is nucleic acid encoding the fusion polypeptide comprising TIR and dimerization domain and said nucleic acid is expressed in the host cells. The field of the invention is nucleic acid encoding fusion polypeptide comprising TIR and dimerization domain or recombinantly produced fusion polypeptide comprising TIR and dimerization domain used for manufacturing a drug for the delivery to the target cells where it acts as an inhibitor in inflammatory conditions resulting from excessive and/or improper TLR/IL-1R family signaling such as inflammatory and autoimmune diseases, sepsis and septic shock, cancer and others. The field of invention is the use of nucleic acid encoding fusion polypeptide comprising TIR and dimerization domain or recombinantly produced fusion polypeptide comprising TIR and dimerization domain for inhibition of cellular TLR/IL-1R family signaling in human or animal cells.

### State of the art

Innate immunity represents the first line of defense against invading microoganisms. An important component of innate immune system represents Toll-like receptors (TLR) which recognize conserved microbial motives called pathogen associated molecular patterns (PAMPs). Ligand binding to the TLR triggers its activation and subsequent recruitment of different adapter proteins into receptor signaling complex. Employing TIR:TIR interactions, adapters connect the TLR with the proteins of downstream signaling cascade mainly including different kinases and ultimately resulting in activation of transcription factors including NF-κB and IRFs leading to inflammatory cytokine and interferon synthesis.

Excessive or non-regulated response to bacterial endotoxin recognized by TLR4 and its co-receptor MD2 on the other hand results in the systemic inflammatory reaction often leading to sepsis as high mortality disease. Furthermore, the development of some chronic inflammatory and autoimmune diseases also arises from the TLR recognition of endogenous ligands resulting in sterile inflammation. Involvement of TLRs has been demonstrated for diseases including arthritis, gouty arthritis, chronic inflammatory bowel disease, psoriasis, multiple sclerosis, ulcerative colitis, Chron's disease, neuropathic pain, alcohol induced inflammation, cancer and others.

For the purpose of ameliorating inflammatory response a selective intracellular TLR inhibition downstream of the receptor can be employed. Methods of selective inhibition of the individual TLR adapters by the use of compounds that decrease their expression/activity by antisense oligonucleotides, siRNA, adaptor specific antibodies or inhibitors binding to them have been proposed thereby inhibiting the TLR pathway corresponding to the selected adapter, e. g. TRAM (U.S. Pat. Appl. Pub. 2005/0158799 A1) or TIRAP (U.S. Pat. 7285535 B2). An antiinflammatory effect can also be achieved by modulation of endogenously present TLR signaling negative regulators such as MyD88s (U.S. Pat. 7122656 B2) or IRAK-M (U.S. Pat. Appl. Pub. 2003/0157539 A1) influencing their expression or activity.

By the means of interfering with protein:protein interactions in the TLR signaling cascade mediated by receptor and adapter TIR domains, frequently involving their BB loops, another principle of the TLR selective inhibition can be achieved. It has been suggested that BB loop peptides and peptidomimetics can occupy the docking site of the cognate adapter on its target and prevent binding of the native adapter thereby disrupting the formation of the functional signaling platform. Peptides and peptidomimetics that mimic a particular portion of MyD88 preventing its homodimerization by interfering with the TIR domain for MyD88 dependent pathways inhibition have been postulated (U.S. Pat. Appl. Pub. 2008/0064643 A1). The selective inhibition of TLR4 signaling can be achieved by the use of peptides corresponding to the BB loops of MyD88, TRAM, TIRAP and TRIF blocking the TLR4 mediated gene expression (Intl. Pat. Appl. Pub. WO 2006/113530 A2). However, the inhibitory potential of the above described peptides and peptidomimetics is relatively weak due to the high concentrations needed for their inhibition. Besides, their use is also restricted to specific TLR pathways, e.g. peptides corresponding to the BB loops of MyD88, TRAM, TIRAP and TRIF influence only TLR4 signaling pathway (Intl. Pat. Appl. Pub. WO 2006/113530 A2) WO 2010/050902 discloses vaccine which consist of fusion polypeptides comprising the TIR domain and a dimerization domain for activating the TLR pathway.

### Summary of the invention

The problem of low activity of peptides interacting with proteins in innate immunity signaling pathway is solved by the use of fusion polypeptides comprising TIR and dimerization domain. We discovered surprisingly that the excessive TLR/IL1-R signaling pathway activation can be restrained by the use of fusion polypeptides comprising TIR domain and dimerization domain which interfere with TIR:TIR interactions between receptors having TIR domains and adapters having TIR domains wherein the said fusion polypeptides in addition to the TIR domain also contain the dimerization domain wherein the two are connected by a linker peptide. The present invention reveals the fusion polypeptides comprising TIR and dimerization domain which have a significantly better inhibitory potential for the TLR/IL-1R family signaling than monomeric inhibitory TIR domains or the BB loop inhibitory peptides and peptidomimetics. The improved inhibitory potential is due to the presence of the domain which enables dimerization of the TIR characterized by SEQ ID NO: 16. Additionally, the fusion polypeptides comprising TIR and dimerization domain are further improved by optionally added segment for membrane localization improving their inhibitory potential. Moreover, the new fusion polypeptides comprising TIR and dimerization domain act specifically on the TLR/IL-1R family signalization by directly interfering with TIR:TIR interactions of the signaling complexes instead of influencing the expression or activity of the individual adapters. Furthermore, the TIR domain contained in the fusion polypeptide comprising TIR and dimerization domain can be chosen among different homologous animal, human or viral TIR domains of TLR signaling adapters, TLRs, IL-1R family members and TIR domain containing viral proteins enabling inhibition of selected TLR/IL-1R family signaling pathways in contrast to the proposed MyD88 homodimerization inhibitors which inhibit only MyD88 dependent pathways (U.S. Pat. Appl. Pub. 2008/0064643 A1) or the BB loop peptides which block only the TLR4 pathway (Intl. Pat. Appl. Pub. WO 2006/113530 A2). The proposed fusion polypeptides comprising TIR and dimerization domain can be used for inhibition of cellular TLR/IL-1R signaling and/or manufacturing a drug for the treatment of chronic and acute diseases resulting from excessive or improper TLR/IL-1R signalization. Nucleic acids encoding fusion polypeptides comprising TIR and dimerization domain can be systemically or locally introduced using different means such as lipofection, microinjection, electroporation, viral delivery etc. Additionally, an improvement in the therapy is the use of fusion polypeptides comprising TIR and dimerization domain as therapeutic agents instead of nucleic acids encoding them thereby avoiding the need for the gene therapy. In this aspect, particulary the fusion polypeptides comprising TIR and dimerization domain additionally containing protein transduction domain are of great importance by enabling the efficient and rapid translocation of the proposed therapeutics into target cells.

The invention refers to fusion polypeptide as defined in the appended claims comprising TIR and dimerization domain which is composed of a) native or mutated homologous animal, human or viral TIR domain of TLR signaling adapters, TLRs, IL-1R family members, TIR domain containing viral proteins, b) dimerization domain and where a) and b) are linked with peptide linker containing flexible and hydrophilic amino acids. Optionally, the fusion polypeptide comprising TIR and dimerization domain also contains amino acid tag and/or a segment enabling membrane localization. The invention refers to TLR signaling pathway inhibitory fusion polypeptide comprising TIR and dimerization domain wherein the TIR domain of the fusion polypeptide comprising TIR and dimerization domain refers to native or mutated homologous animal, human or viral TIR domain of TLR signaling adapters MyD88, MAL, TRIF, TRAM and SARM, TLRs, IL-1R family members, TIR domain containing viral proteins, preferentially TIR domain of signaling adapter MyD88, preferentially SEQ ID NO: 12. The invention refers to TLR signaling pathway fusion polypeptide comprising TIR and dimerization domain wherein the dimerization domain of the fusion polypeptide comprising TIR and dimerization domain is the protein domain which enables spontaneous dimerization of the said fusion polypeptide or the protein domain which enables dimerization of the said fusion polypeptide after addition of chemical ligand which triggers dimerization by binding to these protein domains. The dimerization domain enabling spontaneous dimerization of the TIR domain polypeptide is SEQ ID NO: 16.

The invention refers to fusion poylpeptide as defined in the appended claims comprising TIR and dimerization domain wherein the peptide linker is placed between dimerization domain and TIR domain in the fusion polypeptide comprising TIR and dimerization domain. The peptide linker is from 3 to 40 amino acids long, preferentially from 15 to 30 amino acids, preferentially 25 amino acids long, more preferentially SEQ ID NO: 18. Preferentially amino acids are selected, but not limited to, serine, glycine, threonine, proline, valine, alanine, glutamine, asparagine, glutamate, aspartate residues.

The invention also refers to fusion polypeptide as defined in the appended claims comprising TIR and dimerization domain which in addition to a) native or mutated homologous animal, human or viral TIR domain of TLR signaling adapters, TLRs, IL-1R family members, TIR domain containing viral proteins, b) dimerization domain and where a) and b) are linked with peptide linker containing flexible and hydrophilic amino acids, optionally also containing amino acid tag and/or a segment enabling plasma membrane localization, contains a protein transduction domain for the transduction of the fusion polypeptide into the eukaryotic target cells. The protein transduction domain is selected among cationic peptide sequences, such as PTD of HIV-1 TAT protein, the herpes simplex virus 1 (HSV-1) DNA-binding protein VP22 and the Drosophila Antennapedia (Antp) homeotic transcription factor, preferentially PTD of HIV-1 TAT protein, preferentially SEQ ID NO: 22.

The invention refers to the nucleic acid encoding fusion polypeptide as defined in the appended claims comprising TIR and dimerization domain described above whereas the nucleic acid is operatively linked to the regulatory elements, promoter and terminator to promote expression of the fusion polypeptide comprising TIR and dimerization domain in the host organisms.

Also described is a host cell/organism expressing the above described nucleic acid encoding fusion polypeptide comprising TIR and dimerization domain and to the target cell/organism where the fusion polypeptide comprising TIR and dimerization domain inhibits the TLR/IL-1R signaling pathway.

The invention relates to nucleic acid encoding fusion as defined in the appended claims comprising TIR and dimerization domain which can be used for the inhibition of cellular TLR/IL-1R signaling for the innate immunity research and/or for manufacturing a drug for the treatment of the inflammatory conditions such as inflammatory and autoimmune diseases, sepsis and septic shock, cancer and others resulting from improper and/or excessive TLR signaling in the target cells employing delivery including but not limited to lipofection, microinjection, biolistics, immunolipofection, electroporation, gene bombardment, viral delivery.

The invention also relates to fusion polypeptide comprising TIR and dimerization domain as defined in the appended claims which is produced in, isolated and purified from the host cells/organisms and can be used for the inhibition of cellular TLR/IL-1R signaling for the innate immunity research and/or for manufacturing a drug for the treatment of the inflammatory condition for the treatment of inflammatory conditions, such as inflammatory and autoimmune diseases, sepsis and septic shock and cancer resulting from improper and/or excessive TLR signaling in the target cells/organisms employing delivery via including but not limited to electroporation, microinjection and liposome delivery.

The invention also relates to fusion as defined in the apended claims comprising TIR and dimerization domain which in addition to a) native or mutated homologous animal, human or viral TIR domain of TLR signaling adapters, TLRs, IL-1R family members, TIR domain containing viral proteins, b) dimerization domain placed and where a) and b) are linked with peptide linker optionally also contains amino acid tag and/or a segment enabling plasma membrane localization, contains a protein transduction domain for the transduction of the fusion polypeptide into the eukaryotic target cells, and which is produced in, isolated and purified from the host cells/organisms and can be used for the inhibition of cellular TLR/IL-1R signaling for the innate immunity research and/or for manufacturing a drug for the treatment of the inflammatory conditions such as inflammatory and autoimmune diseases, sepsis, septic shock, cancer and others resulting from improper and/or excessive TLR signaling in the target cells/organisms employing delivery via protein transduction.

### Figure legend

Figure 1. Schematic presentation of fusion polypeptide comprising TIR and dimerization domain. Dimerization of a fusion polypeptide of present invention can be spontaneous (a) or ligand-induced (b).
Figure 2. Constitutive effect of fusion polypeptides comprising TIR and dimerization domain: ccs (TcpB)-TIR MyD88 and GCN-lin-TIR MyD88, each composed of coiled-coil segment, linker peptide and TIR domain on the NF-κB promoter activity on HEK293 cells (a). Improved inhibition of agonist induced TLR4 (b), TLR5 (c) and TLR9 (d) MyD88-dependent signaling pathway by fusion polypeptides comprising TIR and dimerization domain: ccs (TcpB)-TIR MyD88 and/or GCN-lin-TIR MyD88 in comparison with monomeric TIR MyD88 domain.
Figure 3. Constitutive effect of fusion polypeptide containing TIR and dimerization domain: ccs (TcpB)-TIR MyD88 and GCN-lin-TIR MyD88 on the IFNβ promoter activity on HEK293 cells (a). Improved inhibition of agonist induced TLR3 signaling pathway by GCN-lin-TIR MyD88 in comparison with monomeric TIR MyD88 domain (b).
Figure 4. Linker peptide inserted between coiled-coil domain and TIR domain is necessary for efficient inhibition by fusion polypeptide comprising TIR and dimerization domain as demonstrated by increased inhibition of agonist induced TLR4/MD2 signaling with GCN-lin-TIR MyD88 in comparison with GCN-TIR MyD88 which does not contain linker peptide.
Figure 5. Improved inhibition of the agonist induced MyD88 dependent IL-1R signaling by GCN-lin-TIR MyD88 in comparison with monomeric TIR MyD88 domain measured by NF-κB promoter activity on HEK293 cells.
Figure 6. ccs (TcpB)-TIR MyD88 and GCN-lin-TIR MyD88 do not affect the TNFα-R signaling pathway.

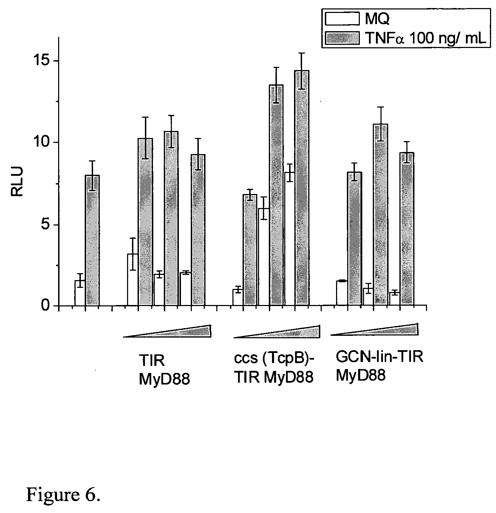

Figure 6. The effect of the added segment for membrane localization myr-TAT to GCN-lin-TIR MyD88 on its constitutive activity (a) and inhibitory potential on agonist induced TLR4/MD2 signaling (b) as determined by NF-κB promoter activity measurement on HEK293 cells.

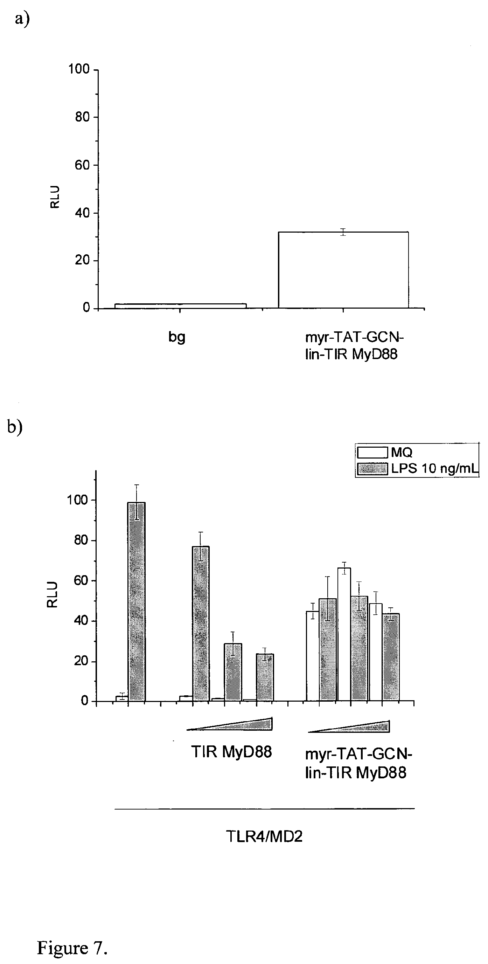

Figure 7. SDS-PAGE of recombinant TAT-GCN-lin-TIR MyD88 polypeptide (a). The inhibitory effect of recombinant self-transducible TAT-GCN-lin-TIR MyD88 on agonist induced TLR5 signaling on HEK293 cells.

### Detailed description

The present invention describes new inhibitors of innate immune Toll-like receptors (TLR)/IL-1R (Interleukin-1 receptor) family members signaling pathways. Invention is based on the discovery, that TLR/IL-1R signaling can be blocked by the use of fusion polypeptides comprising TIR domain and dimerization domain wherein the addition of the domains enabling the dimerization of the described fusion polypeptides ameliorates their inhibitory effect.

Unless defined otherwise, all technical and scientific terms used herein possess the same meaning as it is commonly known to experts in the field of invention. The terminology to be used in the description of the invention has the purpose of description of a particular segment of the invention and has no intention of limiting the invention. All publications mentioned in the description of the invention are listed as references. In the description of the invention and in the claims, the description is in the singular form, but also includes the plural form, what is not specifically highlighted for ease of understanding.

### Fusion polypeptide comprising TIR and dimerization domain

The present invention relates to the fusion polypeptide comprising TIR and dimerization domain. It is known in the field of the invention that TIR domains of the TLR signaling adapters such as MyD88 act inhibitory on TLR signaling (Medzhitov et al., 1998, Mol Cell 2, 253-258). Possible mechanisms of their inhibitory potential for the TLR signaling pathway include their capability of binding to the TLRs thereby preventing the binding of the native adapters and/or TIR domains are also able to bind to the other adapters and thereby sequestering them (Fitzgerald et al., 2001, Nature 413, 78-83).

The authors have come to the discovery that the addition of a domain enabling dimerization to the the TIR domain wherein the domain enabling dimerization and the TIR domain are connected by peptide linker significantly improves the inhibitory potential of TIR domain and consequently the dimeric TIR domains inhibit TLR signaling pathway more effectively than monomeric TIR domains.

The term "fusion polypeptide comprising TIR and dimerization domain " in the description of the invention refers to the fusion polypeptide composed of a) native or mutated homologous animal, human or viral TIR domain of TLR signaling adapters, TLRs, IL-1R family members, TIR domain containing viral proteins, b) dimerization domain and where a) and b) are linked with peptide linker containing flexible and hydrophilic amino acids. Optionally, the fusion polypeptide comprising TIR and dimerization domain also contains amino acid tag and/or a segment enabling membrane localization.. The fusion polypeptide comprising TIR and dimerization domain inhibits TLR signaling pathway by interfering into TIR:TIR domain interactions between receptors and TLR signaling adapters thereby hindering signal transduction.

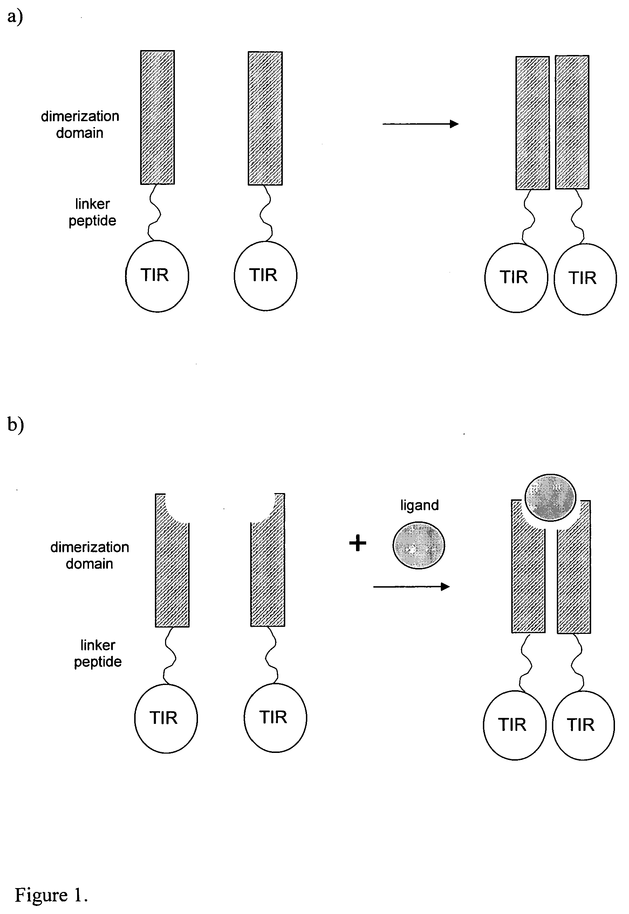

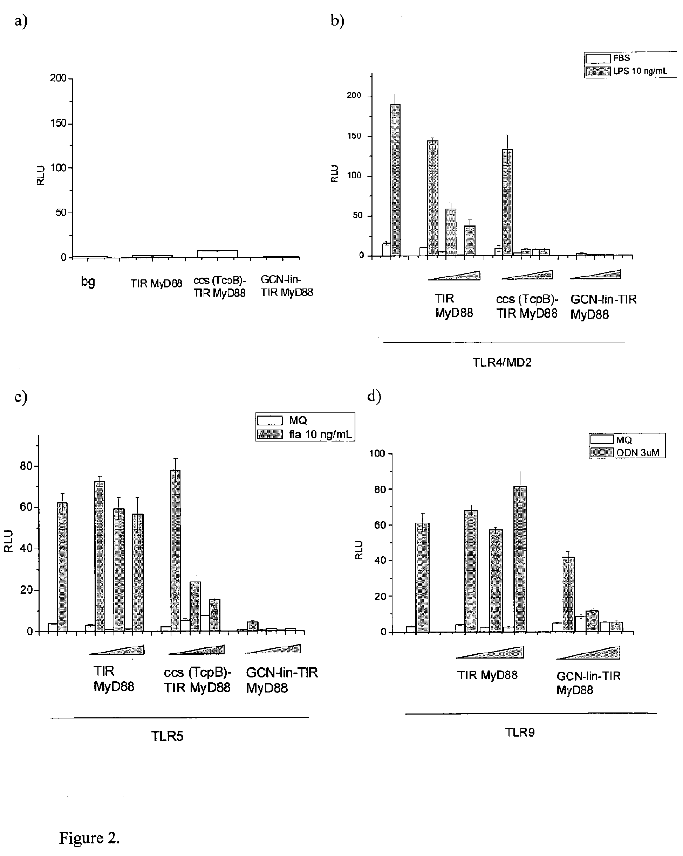

Figure 1 shows schematic presentation of the fusion polypeptide comprising TIR and dimerization domain which can dimerize spontaneous or by the addition of a chemical ligand enabling its dimerization which is described below. On the schematic presentation, the dimerization domain is placed on the N-terminal of TIR domain wherein the two are linked with peptide linker. We have also clearly shown that the addition of dimerization domain on the N-terminal of TIR domain significantly improves the inhibitory potential of monomeric TIR domains on agonist induced TLR signaling (Figures 2 b), c), d), 3 b) and 5) as determined by dual reporter system which is described in the examples of implementation below. The improved inhibitory potential of the addition of dimerization domain on the C-terminal of TIR domain can be easily determined using the same method where the constructs having dimerization domain placed on the C-terminal instead of N-terminal of the TIR domain would be used.

The term "TIR domain" in the description refers to native or mutated homologous animal, human or viral TIR domain of TLR signaling adapters, TLRs, IL-1R family members, TIR domain containing viral proteins which are variable in length and have the sequence conservation above 20 % and all consist of a central five-stranded parallel β-sheet surrounded by five α- helices containing three boxes of conserved residues essential for signalization. TIR domain is besides in TLR signaling adapters also contained in TLRs/IL-1R and is essential for signalization since it connects receptors with proteins in subsequent signaling cascade by the use of adapters.

The term "homologous" refers to the amino acid sequences of protein, originating from the same or another organism, which show a good aminoacid alignment. The term "homologous" refers also to mutant protein segments, whose mutations minimally alter the polypeptide structure and function.

The term "native" refers to the fragment that can be obtained from an organism without prior manipulation of genetic material and the protein or protein fragment is encoded in the genome of this organism.

The term "mutant" refers to the fragment that differs from the native protein/protein fragment in at least one amino acid.

The term "TLR signaling adapters" refers to the cytosolic adapter proteins used in the TLR signaling pathway, namely MyD88, MAL, TRIF, TRAM and SARM. These adapters contain TIR domain which can bind to the TIR domain of TLR and thereby connect the activated TLR with proteins in subsequent cascade by the means of TIR domain. MyD88 participates in all TLR pathways except TLR3 signaling pathway while TRIF participates in TLR3 and TLR4 signaling pathways. TIRAP and TRAM serve as assistant adapters wherein TIRAP connects MyD88 with corresponding receptors in TLR4 and TLR2 signaling pathway and TRAM connects TRIF with the receptor in TLR4 signaling pathway.

The term "Toll-like receptor" refers to any receptor TLR, human or animal. TLR indicates members of the type I transmembrane group of receptors, which consist of a recurrence of the leucine-rich N-terminus, transmembrane region and cytosolic domain that contains the TIR domain. The term refers to native receptors, which have the ability to activate cells or immune response with the addition of appropriate ligands. Toll-like receptors are localized either at the surface of cells (TLR1, TLR2, TLR4, TLR5, TLR6, TLR10, TLR11) or in intracellular vesicles (TLR3, TLR7, TLR8, TLR9). TLR activation occurs with binding of molecules that activate (agonists) TLR. Binding of agonist causes association of cytosolic TIR domains. After the TIR domain association, adapters such as MyD88 or TRIF bind to them and through activation of protein kinase signaling cascade lead to activation of transcription factors and transcripts of genes involved in immune response. Different TLR trigger different responses, either via the MyD88 dependent or TRIF dependent way, and lead to production of pro-inflammatory cytokines such as IL-1, IL-6, TNF-alpha or other interferons.

The term "IL-1 receptor family" refers to receptors of the TIR superfamily with intracellular TIR domain and extracellular immunoglobulin (Ig)-like domain. It includes IL-1 receptor type I (IL-1RI) and its accessory protein (IL-1RAcP), the IL-18 receptors (IL-18Ra and IL-18Rb), the regulatory receptors T1/ST2 and single immunoglobulin IL-1R related molecule (SIGIRR), and of her orphan receptors (TIGIRR-1, TIGIRR-2, and IL-1Rrp2).

The term "TIR domain containing viral proteins" refers to viral proteins containing TIR domains which share amino acid sequence similarity with the mammalian TIR domains. Examples include A46R and A52R proteins from vaccinia virus which are antagonists of host IL-1 and TLR signaling.

Dimerization of fusion polypeptide containing TIR domain and dimerization domain of this invention can be triggered by the dimerization domain which is a part of a fusion polypeptide. Dimerization can be spontaneous or can be triggered with addition of chemical ligand. The basis of the discovery is also that the dimerization domain which causes spontaneous dimerization is preferentially, but not limited to a homodimeric parallel coiled-coil segment which is from 3 to 12 heptads long. The dimerization of the above described fusion polypeptide comprising TIR and dimerization domain can also be triggered by the addition of a small chemical ligand capable of binding to two dimerization domains thereby causing the dimerization of the above described fusion polypeptide comprising TIR and dimerization domain and consists of them. Relevant to ligand induced dimerization, small chemical ligand can be but is not limited to e.g. FK506 which initiates dimerization of FKBP (FK506 binding protein) or cyclosporin which initiates dimerization of cyclophilin.

The term "dimerization domain" refers to the protein domain which enables spontaneous dimerization of the fusion polypeptide comprising TIR and dimerization domain or to the protein domain which enables dimerization of the the fusion polypeptide comprising TIR and dimerization domain after addition of chemical ligand which triggers dimerization by binding to these protein domains. Dimerization domain enabling spontaneous dimerization is preferentially but is not limited to coiled-coil forming segment. Relevant examples of ligand induced dimerization include but are not limited to FK506 which initiates dimerization of FKBP (FK506 binding protein) or cyclosporin which initiates dimerization of cyclophilin.

The term "spontaneous dimerization" in the description refers to the dimerization of the fusion polypeptide comprising TIR and dimerization domain whereby the dimerization domain can dimerize by itself and therefore triggers dimerization of the whole fusion polypeptide comprising TIR and dimerization domain.

The term "ligand-induced dimerization" in the description refers to the dimerization of the fusion polypeptide comprising TIR and dimerization domain caused by the addition of a chemical ligand which binds to two dimerization domains of a fusion polypeptide thereby causing its dimerization.

The term "coiled-coil forming segment" has a general meaning and in the description refers to structural protein motive comprising α-helix which is able to homodimerize with the coiled-coil forming segment of the fusion polypeptide comprising TIR and dimerization domain thereby triggering their dimerization. Coiled-coil forming segments twist around each other forming a super coil. They contain a heptad repeat designated (a-b-c-d-e-f-g)n every two turns of a helix. "a" and "d" usually represent nonpolar, hydrophobic residues that are found at the interface of the two helices, "e" and "g" are solvent exposed polar residues that interact electrostatically, "b", "c" and "f" are hydrophilic and exposed to the solvent. Different amino acids on positions "a-g" define oligomerization state, specify, helix orientation and stability. More specifically, the term coiled-coil segment in the description refers to naturally occurring or designed coiled-coil protein structure motives which comprise from 3 to 12 heptads, are in parallel orientation and can form homodimers. The coiled-coil segments could be selected from naturally occurring or designed coiled-coil protein structure motives, preferentially from naturally occurring, preferentially GCN4-p1, preferentially SEQ ID NO: 16.

The term "homodimerization" in the description of invention has a general meaning and refers to a process of forming dimers composed of only one type of monomers.

The term "monomer" in the above description refers to one coiled-coil forming segment that may interact with the other coiled-coil segment in such manner that they twist around each other.

The term "parallel orientation" has a general meaning and refers to two helices running N to C terminal alongside each other.

The basis of the discovery is also that a peptide linker from 3 to 40 amino acids long should be placed between dimerization domain and TIR domain in the fusion polypeptide comprising TIR and dimerization domain contributing to its efficient inhibition. Optionally, the fusion polypeptide comprising TIR and dimerization domain also contains amino acid tag and/or a segment enabling plasma membrane localization. Namely, the signaling complex involving TLR receptors and adapters is formed on the plasma membrane, often in specialized membrane microdomains, such as lipid rafts. Localization of the fusion polypeptide comprising TIR and dimerization domain to the membrane could therefore contribute to the efficient inhibition due to disruption of the signaling complex formed on the membrane by interfering with TIR:TIR domain interactions of the receptors and adapters important for subsequent signal transduction.

The term "peptide linker" refers to shorter amino acid sequence which is placed between the dimerization domain and the TIR domain assuring their separation and optimal positioning of the TIR domains. The peptide linker is usually from 3 to 40 amino acids long, preferentially from 15 to 30 amino acids, more preferentially it is 25 amino acids long. Preferentially amino acids are selected, but not limited to, serine, glycine, threonine, proline, valine, alanine, glutamine, asparagine, glutamate, aspartate residues that allow flexibility.

The term "tag" refers to short amino acid sequences added to the fusion polypeptide for simplified purification/isolation/detection of the isolated protein.

The term "segment enabling plasma membrane localization" has a general meaning and in the description refers to the amino acid sequence which enables the targeting of the fusion polypeptide comprising TIR and dimerization domain to the membrane. The segment enabling plasma membrane localization can be the amino acid sequence which can be covalently modified by the attachment of the lipid groups which contribute to the membrane-binding affinity of the fusion protein. Relevant example is a myristoylation sequence. In addition, for efficient membrane binding a polybasic cluster of amino acids which forms electrostatic interactions with acidic phospholipid headgroups in the membrane can also represent the segment enabling plasma membrane modification.

Fusion polypeptide comprising TIR and dimerization as defined in the appended claims is encoded by a nucleic acid inserted into an expression vector. The expression vector with inserted nucleic acid encoding fusion polypeptide comprising TIR and dimerization domain is used for inhibition of cellular TLR/IL-1R signaling in human or animal cells and/or for manufacturing a drug for the treatment of diseases resulting from improper and/or excessive TLR/IL-1R signaling, including inflammatory and autoimmune diseases, sepsis, septic shock, cancer and others. The expression vector with inserted nucleic acid encoding fusion polypeptide comprising TIR and dimerization domain and/or the manufactured drug containing the expression vector with inserted nucleic acid encoding fusion polypeptide comprising TIR and dimerization domain is delivered to the host cell/target cell subsequently expressing the nucleic acid encoding fusion polypeptide comprising TIR and dimerization domain. The synthesized fusion polypeptide comprising TIR and dimerization domain in the host cell/target cells is used as an inhibitor of the TLR/I1-1R signaling pathway for treatment of diseases resulting from improper and/or excessive TLR signaling, including inflammatory and autoimmune diseases, sepsis, septic shock, cancer and others. In this embodiment, the nucleic acid coding for the fusion polypeptide comprising TIR and dimerization domain inserted into expression vector and delivered to the host cell/target cell subsequently expressing the nucleic acid encoding fusion polypeptide comprising TIR and dimerization domain, is used for treatment of diseases resulting from improper and/or excessive TLR/IL-1R signaling, including inflammatory and autoimmune diseases, sepsis, septic shock, cancer and others by the means of gene therapy.

In another embodiment, the fusion as defined in the appended claims comprising TIR and dimerization domain encoded by nucleic acid can be synthesized in the host cell/organism and then recombinantly produced, isolated and purified from host cell/organism. The recombinantly produced fusion polypeptide comprising TIR and dimerization domain is used for inhibition of cellular TLR/IL-1R signaling in human or animal cells and/or manufacturing a drug that can then be subsequently delivered to the target cells/organisms where it is used as an inhibitor of the TLR/IL-1R signaling pathway for treatment of diseases resulting from improper and/or excessive TLR/IL-1R signaling, including inflammatory and autoimmune diseases, sepsis, septic shock, cancer and others thereby avoiding the need for a gene therapy with nucleic acids encoding fusion polypeptide of the invention. In this embodiment, the fusion polypeptide comprising TIR and dimerization domain preferentially in addition to a) native or mutated homologous animal, human or viral TIR domain of TLR signaling adapters, TLRs, IL-1R family members, TIR domain containing viral proteins, b) dimerization domain and where a) and b) are linked with peptide linker containing flexible and hydrophilic amino acids, optionally also containing amino acid tag and/or a segment enabling plasma membrane localization also contains a protein transduction domain for the transduction of the fusion polypeptide of the invention into the eukaryotic target cells.

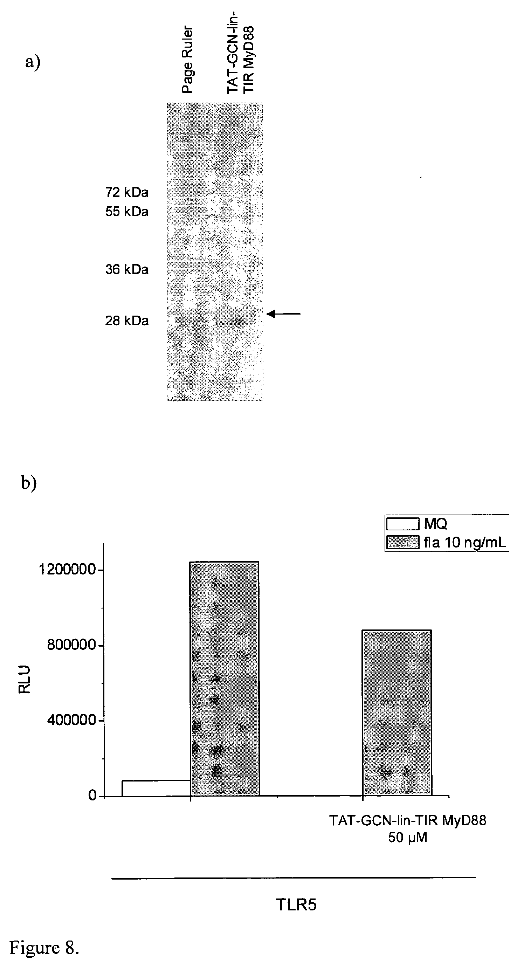

Figure 8 b) represents an example of inhibitory potential of recombinantly produced fusion polypeptide of the present invention with added protein transduction domain according to the invention on flagellin induced TLR5 cell activation which was determined by dual reporter system described in the examples of implementation below. In this case, the protein transduction domain is placed on the N-terminal of dimerization domain of the fusion polypeptide of the present invention. The inhibitory potential of the addition of transduction domain on the C-terminal of TIR domain can be easily determined using the same method where the fusion polypeptide of the present invention having protein transduction domain placed on the C-terminal of TIR domain instead of N-terminal of the dimerization domain would be used.

The term "protein transduction domain (PTD)" has a general meaning and refers to a cationic peptide sequences which enable the intracellular delivery of peptides and proteins. The basic PTD is fused to the inhibitory fusion protein thereby enabling its successful transport through the membrane into the cell. Relevant examples include but are not limited to PTD of HIV-1 TAT protein, the herpes simplex virus 1 (HSV-1) DNA-binding protein VP22 and the Drosophila Antennapedia (Antp) homeotic transcription factor. The advantage of using fusions with protein transduction domain is rapid, concentration-dependent and broad range cell entry and their low toxicity.

### DNA coding for fusion polypeptide comprising TIR and dimerization domain

The present invention also relates to DNA encoding fusion as defined in the appended claims comprising TIR and dimerization domain composed of a) native or mutated homologous animal, human or viral TIR domain of TLR signaling adapters, TLRs, IL-1R family members, TIR domain containing viral proteins, b) dimerization domain and where a) and b) are linked with peptide linker containing flexible and hydrophilic amino acids, optionally also containing amino acid tag and/or a segment enabling plasma membrane localization. The present invention relates also to DNA encoding fusion as defined in the appended claims comprising TIR and dimerization domain which preferentially in addition to a) native or mutated homologous animal, human or viral TIR domain of TLR signaling adapters, TLRs, IL-1R family members, TIR domain containing viral proteins, b) dimerization domain and where a) and b) are linked with peptide linker containing flexible and hydrophilic amino acids, optionally also containing amino acid tag and/or a segment enabling plasma membrane localization, also contains a protein transduction domain.

The term "DNA" in the description refers to a sequence of nucleotides having an open reading frame that encodes a protein of the subject invention, being operatively linked to the regulatory elements, promoter and terminator to promote expression of the protein in the host cells. The length of the DNA sequence may vary greatly depending on the particular protein.

The term "regulatory elements" in the description has a general meaning and refers to the region of DNA in expression vector where regulatory proteins such as transcription factors bind and control gene expression.

The term "promoter" in the description has a general meaning and refers to a region of DNA in expression vector facilitating the transcription of target gene. The type of the promoter depends on host organism in which the gene will be expressed. In case of expression in prokaryotes, namely *E. coli,* several promoters such as Plac, PT5 and preferentially PT7 can be used. In case of expression in eukaryotes, namely in mammalian cells, the promoters are in most cases of viral origin such as PCMV or PSV40.

The term "terminator" in the description has a general meaning and refers to the sequence that marks the end of a gene for transcription. The type of the terminator depends on host organism in which the gene will be expressed. In case of expression in prokaryotes, namely *E. coli,* a terminator from bacteriophage T7 can be used.

Unless otherwise stated, standard molecular biology methods, such as gene cloning, gene multiplication by polymerase, nucleic acid detection, fusion protein construct formation, peptide and protein expression in host cells and similar, were used in the invention. These methods are generally known to experts in the field and are described in detail in different manuals (see Sambrook et al. 1989. Molecular Cloning: A laboratory manual, 2nd ed., Cold Spring Harbor, NY; Ausubel et al. Current Protocols in Molecular Biology, Green Publishing Associates, Inc and John Wiley & Sons, Inc, NY).

Above described fusion polypeptide comprising TIR and dimerization domain is encoded by DNA inserted into an expression vector (viral or non-viral). Fusion polypeptide comprising TIR and dimerization domain, encoded by the nucleic acid according to the invention, can be synthesized in the host cells/organism that expresses the nucleic acid, encoding the fusion polypeptide, from the expression vector according to the invention. The synthesized fusion polypeptide comprising TIR and dimerization domain is in the target cell used for the inhibition of the TLR/IL-1R signaling pathway and/or for the treatment of diseases resulting from improper and/or excessive TLR signaling, including inflammatory and autoimmune diseases, sepsis, septic shock, cancer and others.

Suitable vectors for the expression of DNA encoding fusion polypeptide comprising TIR and dimerization domain include, but are not limited to: plasmids, viral vectors, and others. Expression vectors, compatible with host organism cells, are well known to experts in the field and contain appropriate control elements for transcription and translation of nucleic acids. Typically an expression vector includes an expression cassette, composed in the direction 5' to 3' of a promoter, the fusion protein coding sequence operationally linked with the promoter and a terminator including a stop codon for RNA polymerase and a polyadenylation signal for the polyadenylase.

In an embodiment, where the DNA encoding fusion polypeptide comprising TIR and dimerization domain will be used for inhibition of cellular TLR/IL-1R signaling in human or animal cells and/or manufacturing a drug which will eventually be used for gene therapy for treatment of diseases resulting from improper and/or excessive TLR signaling, including inflammatory and autoimmune diseases, sepsis, septic shock, cancer and others, an expression vector should be prepared for expression in eukaryotic cells. According to the invention the use of prokaryotic cells is intended for preparation of a sufficient quantity of the nucleic acid. Eukaryotic expression vectors are used to express the DNA in eukaryotes.

In an embodiment, where instead of the DNA encoding fusion polypeptide comprising TIR and dimerization domain, the recombinantly produced fusion polypeptide comprising TIR and dimerization domain will be used for inhibition of cellular TLR/IL-1R signaling in human or animal cells and/or manufacturing a drug and delivered to the target cells/organisms for treatment of diseases resulting from improper and/or excessive TLR signaling, including inflammatory and autoimmune diseases, sepsis, septic shock, cancer and others, an expression vector should be prepared for expression in prokaryotic or eukaryotic cells. Eukaryotic expression vectors are used to express the DNA in eukaryotes, prokaryotic expression vectors are used to express the DNA in prokaryotes.

An expression vector generally contains operationally linked control elements, which are operationally linked to the DNA coding for the fusion polypeptide comprising TIR and dimerization domain according to the invention. It is understood that the control elements are selected according to desired expression quantity and tissue-specific expression. The promoter may be either constitutive or inducible depending on the desired pattern of expression. The promoter may be of either native origin or foreign origin (not presented in the cells, where it is applicable), and may be natural or synthetic. The promoter is chosen in such a way that it functions in the target cells of the host organism. In addition, initiation signals for the efficient translation of the fusion protein, including the ATG and corresponding sequences, are included. In the case when the vector used in the invention includes two or more reading frames, these should be operationally linked with control elements independently where the control elements should be either equal or different, depending on the desired protein production.

Examples of bacterial expression vectors include, but are not limited to: pET vectors, pRSET vectors, and others. When vectors are used in the bacterial cells, the control elements are of bacterial origin.

Examples of mammalian expression vectors for mammalian cells include, but are not limited to: pcDNA (Invitrogen), pFLAG (Sigma), and others. When vectors are used in mammalian cells, the control elements are in most cases of viral origin, for example, adenovirus 2, cytomegalovirus, a virus Simian virus 40.

### Host cell/organism/ Target cell/organism

Also described is a host cell/organism expressing the above described DNA encoding fusion polypeptide of the invention and to the target cell/organism where the fusion polypeptide comprising TIR and dimerization domain inhibits the TLR/IL-1R signaling pathway.

The term "host cell/organism" refers to the cell/organism, in which the DNA coding for fusion polypeptide comprising TIR and dimerization domain has been introduced in order to be expressed. The expression of DNA encoding fusion polypeptide comprising TIR and dimerization domain results in its synthesis in the host cell/organism.

In an embodiment, where the DNA encoding fusion polypeptide comprising TIR and dimerization domain will be used for used for inhibition of cellular TLR/IL-1R signaling in human or animal cells and/or manufacturing a drug which will eventually be used for gene therapy for treatment of diseases resulting from improper and/or excessive TLR signaling, including inflammatory and autoimmune diseases, sepsis, septic shock, cancer and others, the host cell/organism also represents the target cell/organism since the fusion polypeptide comprising TIR and dimerization domain encoded by DNA is not only introduced and synthesized but also inhibits TLR/IL-1R signaling pathway in the host cell/organism. In this embodiment, the host cell/target cells are animal, preferentially vertebrate, more preferentially mammalian cells, or human cells or organisms. Methods of the DNA encoding fusion polypeptide comprising TIR and dimerization domain delivery to the host/target cells are in more detail described below.

In an embodiment where instead of the DNA encoding fusion polypeptide comprising TIR and dimerization domain, the recombinantly produced fusion polypeptide comprising TIR and dimerization domain will be used for inhibition of cellular TLR/IL-1R signaling in human or animal cells and/or manufacturing a drug and delivered to the target cells/organisms for treatment of diseases resulting from improper and/or excessive TLR/IL-1R signaling, including inflammatory and autoimmune diseases, sepsis, septic shock, cancer and others, the host cell/organism may be prokaryotic or eukaryotic. Eukaryotic cells, suitable for the expression of the fusion polypeptide of the present invention, are not limited as long as cell lines are compatible with propagation methods of the expression vector and with the expression of the fusion protein. The preferred eukaryotic cells include, but are not limited to, yeast, fungi, plant cells and mammalian cells, such as: mouse, rat, monkey or human fibroblasts. For the DNA expression any bacterial host can be used according to the invention. For the expression of polypeptide of the present invention, the use of bacteria or yeast is preferred. The polypeptide can be expressed in bacteria *E. coli* or *B. subtilis* or in yeast *S*. *cerevisiae* or *P. pastoris.* The preferred bacterium is *E. coli.* Invention refers to the expression of the polypeptide in bacteria or yeast. Invention refers to bacteria or yeast, that express polypeptide, preferably to the bacteria *E*. *coil* or to yeast *P. pastoris.*

The transfer of expression vector with DNA encoding fusion polypeptide comprising TIR and dimerization domain into host cell/organism is carried out by conventional methods, known to the experts from the field, and the methods refer to transformation, transfection, including: chemical transfer, electroporation, microinjection, DNA lipofection, cell sonication, particle bombardment, viral DNA transfer, and more.

The term "target cell/organism" refers to the cell/organism in which the inhibition of the TLR/IL1-R signaling will occur as a consequence of delivery and expression of the nucleic acid encoding fusion polypeptide comprising TIR and dimerization domain or the delivery of recombinantly produced fusion polypeptide comprising TIR and dimerization domain to the target cell/organism for the innate immune system research or for the treatment of diseases resulting from improper and/or excessive TLR signaling, including inflammatory and autoimmune diseases, sepsis, septic shock, cancer and others. The target cells/organisms are animal, preferentially vertebrate, more preferentially mammalian cells, or human cells or organisms.

In an embodiment, where the DNA encoding fusion polypeptide comprising TIR and dimerization domain will be used for the inhibition of the TLR/IL1-R signaling and/or manufacturing a drug which will eventually be used for gene therapy for treatment of diseases resulting from improper and/or excessive TLR signaling, including inflammatory and autoimmune diseases, sepsis, septic shock, cancer and others, the DNA encoding fusion polypeptide comprising TIR and dimerization domain is introduced and expressed in the target cell and the synthesized fusion polypeptide comprising TIR and dimerization domain inhibits TLR/IL-1R signaling pathway. In another embodiment instead of the DNA encoding fusion polypeptide comprising TIR and dimerization domain, the recombinantly produced fusion polypeptide comprising TIR and dimerization domain is produced in, isolated and purified from the appropriate host cell/organism defined above and will be used for the inhibition of the TLR/IL1-R signaling and/or manufacturing a drug and delivered to the target cells/organisms for treatment of diseases resulting from improper and/or excessive TLR/IL-1R signaling, including inflammatory and autoimmune diseases, sepsis, septic shock, cancer and others.

Methods of DNA encoding fusion polypeptide comprising TIR and dimerization domain and recombinantly produced fusion polypeptide comprising TIR and dimerization domain delivery to the target cells/organisms are described below.

### Recombinant production of fusion polypeptide comprising TIR and dimerization domain

Also described is the recombinant production of the fusion polypeptide comprising TIR and dimerization domain and includes introducing DNA encoding fusion polypeptide comprising TIR and dimerization domain into the host organism, cultivating host organism under conditions suitable for protein expression and isolation and purification of protein.

The fusion polypeptide comprising TIR and dimerization domain can be produced in, isolated and purified from the host organism that expresses DNA which encodes for the fusion polypeptide comprising TIR and dimerization domain. The fusion polypeptide comprising TIR and dimerization domain is used for inhibition of TLR/IL-1R signaling in human or animal cells and/or for manufacturing a drug which is delivered to the target cells/organisms for treatment of diseases resulting from improper and/or excessive TLR signaling, including inflammatory and autoimmune diseases, sepsis, septic shock, cancer and others.

In general, the DNA encoding fusion polypeptide comprising TIR and dimerization domain is incorporated into an expression vector (viral or non-viral), which is described above. Also described are host cells and organisms that contain DNA according to the invention (transient or stable), which codes for the fusion polypeptide according to the invention. Appropriate host organisms are known to the experts in the field of molecular biology and include prokaryotic and eukaryotic cells which is described above.

The transfer of vectors into host cells is carried out by conventional methods, known in the state of the art, and described above.

The DNA transfer may be transient or stable. Transient expression refers to the introduction of the vector DNA, which according to the invention is not incorporated into the genome of cells. Stable intake is achieved by incorporating DNA of the invention into the host genome. The transfer of the DNA according to the invention, especially for the preparation of the host organism, which has a stable DNA integrated, may according to the invention be controlled by the presence of markers. DNA coding for markers refers to resistance to drugs, for example antibiotics, and may be included in the vector with the DNA according to the invention, or on a separate vector.

The fusion polypeptide comprising TIR and dimerization domain is expressed in the suitable host organism. Expression of large amounts of fusion proteins can be achieved in bacteria such as *E. coli,* in yeast *P. pastoris,* however it is also possible to use mammalian cell culture to produce smaller amounts of proteins when posttranslational modifications are necessary for correct folding and function. It is known that the protein can be expressed in mammalian cells of the following organisms: human, rodents, cattle, pig, poultry, rabbits and alike. The host cells can be cultivated primary cell lines or immortalized cell lines. Expression of fusion polypeptide comprising TIR and dimerization domain can be constitutive or inducible, e.g. expression of protein, can be induced by addition of inducer IPTG, which by binding lac repressor turns on expression of protein of interest. Fusion polypeptide comprising TIR and dimerization domain can be expressed either in soluble fraction or inclusion bodies.

Protein purification techniques including size-exclusion chromatography, ion-exchange chromatography, reverse-phase chromatography, affinity chromatography when fusion proteins or appended tags interact with purification column specifically etc. are well know to the experts in the field.

### Methods of delivery to the target cells/organisms

The invention refers to the inhibition of the TLR/IL-1R signaling pathway in the target cell/organism by the means of administering to the cell an inhibitory amount of DNA encoding the fusion polypeptide comprising TIR and dimerization domain inserted into expression vector. In this embodiment of the invention, DNA encoding fusion polypeptide comprising TIR and dimerization domain can be inserted into expression vectors described above for the *in vitro* or *in vivo* transfection of the target cells through the interaction of a vector and the target cells. The fusion polypeptide comprising TIR and dimerization domain is then expressed from the DNA encoding it.

The introduction of nucleic acid coding for the fusion polypeptide according to the invention into the target cells/organisms can be carried out by various methods from the non-viral delivery including lipofection, microinjection, biolistics, immunolipofection, electroporation, gene bombardment to the viral delivery where the nucleic acid is inserted into DNA or RNA viruses that infect target cells and have episomal or integrated genomes after the delivery to the target cell. Viral-based systems could include retroviral, lentiviral, adenoviral, adeno-associated and herpes-simplex virus vectors for gene transfer. Viral vectors used are usually generated by a producer cell line that packages a nucleic acid vector into a viral particle. The vectors typically contain the minimal viral sequences required for packaging and subsequent integration into a host and an expression cassette for the protein to be expressed. A viral vector can be modified to have specifity for the cell type of interest by expressing a ligand as a fusion protein with a viral coat protein wherein a ligand has an affinity for a receptor of the cell type of the interest. In all the above described methods of the introduction the Fusion polypeptide comprising TIR and dimerization domain is produced from the nucleic acid encoding it in the target cells, where it subsequently acts as a TLR signaling pathway inhibitor.

The nucleic acids can be administered for *in vivo* or *ex vivo* uses. *Ex vivo* administration includes collecting a sample of cells from the individual, for example immune cells, endothelian cells or any other kind of cells for which treatment is beneficial, culturing the collected cells *ex vivo* and adding the nucleic acids to the cells by one of the method described above. After exposure to the nucleic acids the treated cells may be reintroduced into the individual thereby providing prophylactic or therapeutic treatment. *In vivo* therapy use include administering the nucleic acid directly to the individual by systemic administration (e.g. intravenous, intraperitoneal, intramuscular, subdermal) or topical application where the complex is administered directly to a tissue site in the body. The nucleic acids are administered in any suitable manner preferably with pharmaceutically acceptable carriers.

The insertion of DNA may be transitory or stable. By the use of transitory DNA introduction, a DNA is introduced by the means of a vector that does not incorporate the DNA into the genome of the target cells. Stable insertion is enabled by incorporating the invented DNA into the target cell genome, e.g. by the use of viruses (e.g. the retrovirus, lentivirus and adeno-associated virus) which assure the integration of the nucleic acid coding for the fusion polypeptide comprising TIR and dimerization domain into the target cell genome.

For an overview of gene therapy, see Chapter 20, Gene Therapy and other Genetics-based Therapeutic Approaches in Human Molecular Genetics, T. Strachan and A. P. Read, BIOS Scientific Publishers Ltd (1996).

In another embodiment, the fusion polypeptide comprising TIR and dimerization domain which is produced in, isolated and purified from host cells/organisms described above instead of the DNA encoding fusion polypeptide comprising TIR and dimerization domain will be used for delivery to the target cells and inhibition of the TLR/IL1-R signaling and/or for manufacturing a drug which is delivered to the target cells/organisms for treatment of diseases resulting from improper and/or excessive TLR signaling, including inflammatory and autoimmune diseases, sepsis, septic shock, cancer and others.

Fusion polypeptide comprising TIR and dimerization domain which is produced in, isolated and purified from host cells/organisms described above preferably also contains the PTD which enables the intracellular delivery of the fusion polypeptide comprising TIR and dimerization domain to the target cell/organism. Fusion polypeptide comprising TIR and dimerization domain which does not contain PTD can be the delivered into the target cells/organisms employing methods including but not limited to electroporation, microinjection and liposome delivery.

### Fusion polypeptide comprising TIR and dimerization domain for inhibition of cellular TLR/IL-1R signaling and for manufacturing a drug for the treatment of diseases

The present invention refers to the fusion polypeptide as described in the appended claims comprising TIR and dimerization domain which can be used for the inhibition of cellular TLR/IL-1R signaling for the innate immunity research and/or for manufacturing a drug for the treatment of the inflammatory condition in an individual by means of administering manufactured drug consisting of a therapeutically effective amount of the nucleic acid encoding fusion polypeptide comprising TIR and dimerization domain or recombinantly produced fusion polypeptide comprising TIR and dimerization domain which is produced in, isolated and purified from host cells/organisms in combination with a pharmaceutically acceptable carrier using the methods of delivery to the target cells/organisms described above. The fusion polypeptide of the invention inhibits TLR/IL-1R signaling pathway by interfering with TIR:TIR interactions of the TLR signaling complex thereby causing an anti-inflammatory and immunoinhibitory effect in the target cell/organism. The manufactured drug can be used for treatment of inflammatory conditions, such as inflammatory and autoimmune diseases, sepsis, septic shock, cancer and others which are in more detail described below.

The term "manufacturing a drug" refers to a process of a preparation of pharmaceutical compositions which are described below. The term "manufactured drug" refers to pharmaceutical composition described below comprising a therapeutically effective amount of the nucleic acid encoding fusion polypeptide comprising TIR and dimerization domain or of a recombinantly produced fusion polypeptide comprising TIR and dimerization domain in combination with a pharmaceutically acceptable carrier.

Also described are both prophylactic and therapeutic methods of treating target organism at risk of or susceptible to a disorder or having a disorder associated with excessive TLR signaling. The term "treatment" is defined as the delivery of manufactured drug described above to the target organism who has a disease, a symptom of a disease or a predisposition toward the disease with the purpose to cure, alleviate, remedy, ameliorate or improve the disease, a symptom of a disease or a predisposition toward disease. A therapeutically effective amount of DNA coding for fusion polypeptide comprising TIR and dimerization domain or fusion polypeptide of the present invention in combination with a pharmaceutically acceptable carrier is administered to a target cell/organism by the methods of delivery described above with a pharmaceutically acceptable carrier described below.

Also described are methods for using manufactured drug described above for the treatment or amelioration of conditions treatable by inhibiting TLR/IL-1R signaling or TLR/IL-1R-mediated gene expression in an individual. The term "conditions" refers to physiological conditions resulting from excessive TLR signalization including inflammation. More specifically, the invention refers to the treatment of target organisms with the manufactured drug according to the invention with the purpose of treatment and prevention of inflammatory and autoimmune diseases, sepsis, septic shock, cancer and others in more detail described below.

The manufactured drug described above can be used to treat variety of immune disorders deriving from the excessive or improper TLR/IL-1R signaling. Examples include inflammatory inflammatory conditions, such as bacterial, viral, fungal protozoal infectious diseases, sepsis and septic shock, autoimmune diseases such as systemic lupus erythematosis, rheumatoid arthritis, Crohn's disease, inflammatory bowel disease, ulcerative colitis, insulin resistance, cancer, neuropathic pain, neuroinflammation, alcohol induced inflammation, ischaemia, hypoxia, haemorrhage, atherosclerosis, asthma, allergy, graft rejection, akylous spondilytis, gouty inflammation.

The manufactured drug described above can be used to treat or prevent inflammatory responses caused by a microbe (or a toxin from microbe), e.g. a bacterium (e.g a Gram-positive or a Gram-negative bacterium) such as *E. coli, Salmonella typhimurium* or other *Salmonella* species; *Pseudomonas* species or the bacterium can be gram-positive such as species *Staphylococcus, Streptococcus, Pneumococcus.* Other example of microbial infections that can be treated by manufactured drug described above include rickettsia; a virus e.g. Ebola virus, West Nile encephalitis virus and hepatitis a, b, c; fungi e.g. *Candida albicans, Cryptococcus neoformans* and protozoans, e.g. *Plasmodium falciparum* and other species of *Plasmodium* that cause malaria.

The manufactured drug described above can be used to treat or prevent inflammatory reactions triggered by toxins such as any toxin produced by a microbe that causes an inflammatory response for example lipopolysaccharide or any superantigen produced by a microbe, e.g. *Staphylococcus* enterotoxin A or B.

The manufactured drug described above can be used to treat or prevent any inflammatory reactions that can be treated by the methods of the invention including plant toxins, nickel, latex, environmental toxins or allergens that invoke an inflammatory response upon skin contact or inhalation.

The manufactured drug described above can be used to treat both systemic and localized inflammatory responses. The methods and manufactured drug of the invention can be used to treat or prevent inflammatory responses that affect the function of specific organs or organ systems, for example, the liver, bowel, kidney, joints, skin, central nervous system, bladder or reproductive organs. The methods and manufactured drug can also be used to treat chronic or acute inflammatory diseases and conditions of the skin, e.g. psoriasis, eczema or contact dermatitis.

### Pharmaceutical composition

The present invention provides for pharmaceutical compositions comprising a therapeutically effective amount of the nucleic acid encoding fusion polypeptide comprising TIR and dimerization domain or recombinantly produced fusion polypeptide comprising TIR and dimerization domain in combination with a pharmaceutically acceptable carrier including saline, buffered saline, dextrose, water, glycerol, stabilizing compounds, solvents, salts, lubricants, osmostabilizers and others. The term "pharmaceutically acceptable" refers to the material that is not toxic to the host cell/organism. A pharmaceutical composition should to be compatible with its intended route of administration. Examples of route include parenteral (intravenal, intradermal, subcutaneous), oral, inhalation, topical, transmucal.

In an embodiment involving the use of the nucleic acid encoding fusion polypeptide comprising TIR and dimerization domain for the treatment of autoimmune, chronic inflammatory disease, sepsis, septic shock, cancer and others by the means of gene therapy, the pharmaceutical composition is preferentially formulated for viral administration or by any other DNA delivery methods to the target cells/organisms described above.

In an embodiment involving the use of recombinantly produced fusion polypeptide comprising TIR and dimerization domain which is produced in, isolated and purified from host cells/organisms, for the treatment of autoimmune, chronic inflammatory disease, sepsis, septic shock, cancer and others preferentially the pharmaceutical composition is formulated but not limited to the administration via transduction since the fusion polypeptide comprising TIR and dimerization domain can contain the PTD enabling efficient and rapid translocation to the target cells/organisms.

In the context of invention, the nucleic acids coding for fusion polypeptide comprising TIR and dimerization domain or recombinantly produced fusion polypeptide comprising TIR and dimerization domain in combination with a pharmaceutically acceptable carrier can be administered to a target cell/organism at therapeutically effective doses to prevent, treat or ameliorate disorders associated with excessive or improper TLR/IL-1R signaling wherein a therapeutically effective dose refers to the amount of nucleic acid or recombinantly produced fusion polypeptide of the present invention described above sufficient to initiate the inhibition of the TLR/IL-1R signaling pathway amelioration of symptoms of the disorders. Optionally, the delivered quantity is sufficient, when an amelioration of the symptoms of the diseases resulting from excessive or improper TLR/IL-1R signalization is initiated. The inhibition is not necessarily complete and permanent, as long as the benefits of the administration of pharmaceutical mixture outweigh the unwanted effects. Therapeutically effective dose depends on the mode of administration, on the nucleic acid coding for fusion polypeptide comprising TIR and dimerization domain or the recombinantly produced fusion polypeptide according to the invention, on the efficient expression of the protein in case of gene therapy, and on the subject. Effective doses are determined in a manner known in the state of the art.

Examples of implementation, designed to illustrate the invention, are shown below. The descriptions of examples of implementation have no intention of limiting the invention and should be understood as a demonstration of the invention.

### Examples of implementation

### Example 1: DNA construct preparation.

The constructs listed below (Table 1, Table 2) were prepared with the intention of demonstrating the effect of the invention. Fusion polypeptides comprising TIR and dimerization domain according to this invention were designed by combining TIR domain, dimerization domains, linker peptide and membrane localization segment. Amino acid sequence of TIR domain was selected from the TIR domain of adapter MyD88 (SEQ ID NO: 12). Dimerization segments were selected from the GCN4 peptide (SEQ ID NO: 16) which forms homodimeric parallel coiled-coil and from the N-terminal segment (aa 1-117) of the TcpB protein from *B. melitensis* for which the dimerization domain comprising homodimeric coiled-coil formation was predicted (ccs (TcpB); SEQ ID NO: 14). The linker peptide was selected from 29 flexible and hydrophilic amino acids long peptide (SEQ ID NO: 18). The membrane localization segment was selected from myristoilation sequence from adapter TRAM (SEQ ID NO: 20) and basic segment TAT (SEQ ID NO: 22). The fusion proteins may contain a signal sequence to guide protein trafficking and a peptide tag for detection or purification of the protein.

DNA constructs and corresponding fusion proteins are described in Table 1. Detailed description and function of individual DNA or protein product are described in Table 2. DNA sequences for segments GCN, lin and TAT were custom made by GeneArt company (http://www.geneart.com/). The DNA constructs were cloned into pcDNA3 vector (http://www.lablife.org//p?a=vdb view&id=g2.9Qyh n9D3As.29eaY9eZRAcswvk-) for expression in mammalian cells, the TAT-GCN-lin-TIR MyD88 construct was cloned into pET3a vector for expression in *E. coli.* The pDeNy-hMyD88 containing the gene for TIR MyD88 was purchased from Invivogen (httn://www.invivogen.com/PDF/pDeNy-hMyD88 TDS.pdf).

DNA constructs were prepared using methods of molecular biology that are basically described in the molecular biology handbook (Sambrook J., Fritsch E.F., Maniatis T. 1989. Molecular cloning: A laboratory manual. 2nd ed. New York, Cold Spring Harbor Laboratory Press: 1659 str.). Plasmids and constructs were transformed into bacterium *E. coli* DH5a. Plasmids for transfection into the cell line HEK293 were isolated using PureLink HiPure Plasmid Kit (InVitrogen) which assures the endotoxin free plasmid isolation.

The final constructs are listed in Table 1 and were prepared with the techniques described above. Nucleotide sequence was confirmed by sequencing reactions. Single parts of the fusion protein, encoded by the DNA, are listed in Table 2 which also contains the sequences of the boundaries of the used parts of the genes. Similarly, for the promoter and terminator, the database that contains the complete sequence and the borders of the nucleotide sequence used are indicated.

**Table 1: Fusion polypeptides, which were used for the demonstration of the invention**

| No. | Name | Construct composition | Nucleotide/ aminoacid sequence | plasmid backbone |
|---|---|---|---|---|
| 1 | ccs (TcpB)-TIR MyD88 | FLAG- ccs (TcpB)-TIR MyD88 | SEQ ID NO: 1 / SEQ ID NO: 2 | pcDNA3 |
| 2 | GCN-TIR MyD88 | GCN-TIR MyD88 | SEQ ID NO: 3 / SEQ ID NO: 4 | pcDNA3 |
| 3 | GCN-lin-TIR MyD88 | GCN-lin-TIR MyD88 | SEQ ID NO: 5 / SEQ ID NO: 6 | pcDNA3 |
| 4 | myr-TAT-GCN-lin-TIR MyD88 | myr-TAT-GCN-lin-TIR MyD88 | SEQ ID NO: 7 / SEQ ID NO: 8 | pcDNA3 |
| 5 | TAT-GCN-lin-TIR MyD88 | His-GG-TAT-GG-GCN-lin-TIR MyD88 | SEQ ID NO: 9/ SEQ ID NO: 10 | pET3a |

**Table 2: Genes, their function and amino acids/nucleotide sequence, which represents the borders of the used parts of genes.**

| Gene name | Nucleotide sequence | Amino acid sequence | function |
|---|---|---|---|
| HIStag | | HHHHHH | tag |
| FLAG_{tag} | | DYKDDDDK | tag |
| GG | GGTGGT | GG | dipeptide linker |
| TIR MyD88 | SEQ ID NO: 11 | SEQ ID NO: 12 | TIR domain of the adapter MyD88 |
| ccs (TcpB) | SEQ ID NO: 13 | SEQ ID NO: 14 | N-terminal segment of TcpB that is predicted to form homodimeric coiled-coil |
| GCN | SEQ ID NO: 15 | SEQ ID NO: 16 | GCN4-p1 that forms homodimeric parallel coiled-coil |
| lin | SEQ ID NO: 17 | SEQ ID NO: 18 | linker peptide |
| myr | SEQ ID NO: 19 | SEQ ID NO: 20 | myristoilation sequence of adapter TRAM |
| TAT | SEQ ID NO: 21 | SEQ ID NO: 22 | basic segment for protein transduction |

### Example 2. The effect of fusion polypeptides comprising TIR and dimerization domain on TLR/IL-1R and TNFα-R signalization

The effect of the fusion polypeptides comprising TIR and dimerization domain according to the invention on TLR/IL-1R and TNFα-R signalization was demonstrated by transfection of nucleic acid encoding fusion polypeptides of the present invention into the HEK293 cell line and activation of a reporter system, reflecting the activation of the NF-κB or IFNβ promoter.

Methods and techniques of cultivation of cell lines are well-known to experts in the field and are explained here only indicatively, with the intention of clarifying the example of implementation. Cell cultures of HEK293 cells were grown at 37 °C and 5% CO2. For the cultivation, DMEM medium with 10% FBS, containing all the necessary nutrients and growth factors was used. When the cell population reached a sufficient density, the cells were transferred to a new flask and/or diluted. For use in experiments, the number of cells was determined by the use of a hemocytometer and a 96-well microtiter plate, suitable for growing cell cultures, was inoculated with 2,5*10⁴ cells per well 24 hours prior transfection. Inoculated plates were incubated at 37 °C and 5% CO₂ until the cells were 50-70% confluent for the transfection using transfection reagent JetPei. The transfection was carried out according to the manual provided by the manufacturer, adapted to the use of 96-well microtiter plates.

### Dual luciferase assay

Cell activation and inhibition by the fusion polypeptides of the invention were monitored using the dual luciferase assay based on reporter plasmids. For luciferase activity determination, a test with two reporter proteins was used: (a) firefly luciferase (Fluc) and (b) Renilla luciferase (Rluc). The gene for firefly luciferase (Fluc) coding for firefly luciferase, which uses luciferin as substrate, is functionally linked to NF-κB (NF-κB -Fluc) or IFNβ (IFNβ-fluc) promoter, sensing their activation. Activation of TLR leads to activation of NF-κB and/or IFNβ transcription factors, which can thus be detected by measuring the activity of the firefly luciferase. Another reporter, transfected into the cells simultaneously with the plasmid pFluc and plasmids, encoding the fusion polypeptides comprising TIR and dimerization domain, serves as a transfection efficiency reporter. The reporter plasmid bears the DNA coding for Renilla luciferase (Rluc), which uses coelenterazine as a substrate. Rluc is expressed constitutively and is used for normalization of cell transfection efficiency.

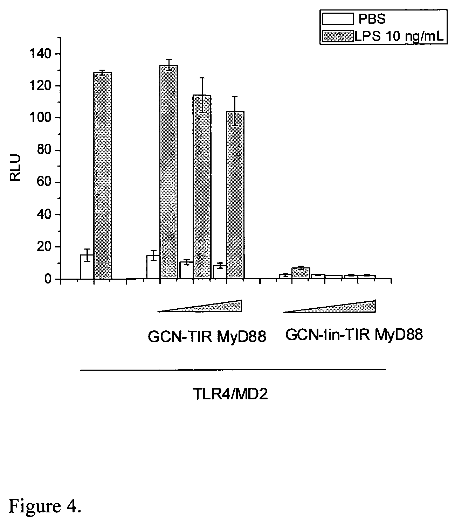

For the transfection of the cell line HEK293 the DNA plasmids encoding fusion polypeptides of the invention listed in Table 1, different TLR and two reporters: Rluc and NF-κB -Fluc/IFNβ-Fluc were used in the following concentrations:
Figure 2
   a) For determining the constitutive effect of fusion polypeptides comprising TIR and dimerization domain on the NF-κB promoter activity: 10 ng Rluc; 50 ng NF-κB -Fluc; 140 ng of the investigated plasmid
      For determining the inhibitory effect of the fusion polypeptides comprising TIR and dimerization domain on ligand-induced TLR cell activation in comparison with TIR domain:
   b) On TLR4/MD2 signaling: 1 ng TLR4, 1 ng MD2, 10 ng Rluc; 50 ng NF-κB -Fluc; 1 ng, 10 ng and 20 ng of the investigated plasmid
   c) On the TLR5 signaling: 30 ng TLR5; 10 ng Rluc; 50 ng NF-κB -Fluc, 1 ng, 10 ng and 20 ng of the investigated plasmid
   d) On the TLR9 signaling: 40 ng TLR9; 5 ng Unc93B1, 10 ng Rluc, 100 ng NF-κB - Fluc; 1 ng, 10 ng and 20 ng of the investigated plasmid
Figure 3.
   a) For determining the constitutive effect of fusion polypeptides comprising TIR and dimerization domain on the IFNβ-Fluc promoter activity: 10 ng Rluc; 40 ng IFNβ-fluc; 100 ng of the investigated plasmid.
   b) For determining the inhibitory effect of the fusion polypeptides comprising TIR and dimerization domain on ligand-induced TLR cell activation in comparison with TIR domain on TLR3 signaling: TLR3 20 ng; 10 ng Rluc; 40 ng IFNβ-fluc; 1ng, 10 ng and 20 ng of the investigated plasmid
Figure 4. For comparing the inhibitory potential of fusion polypeptides comprising TIR and dimerization domain with or without linker peptide on ligand-induced TLR4/MD2 signaling: 1 ng TLR4, 1 ng MD2, 10 ng Rluc; 50 ng NF-κB -Fluc; 1 ng, 10 ng and 20 ng of the investigated plasmid

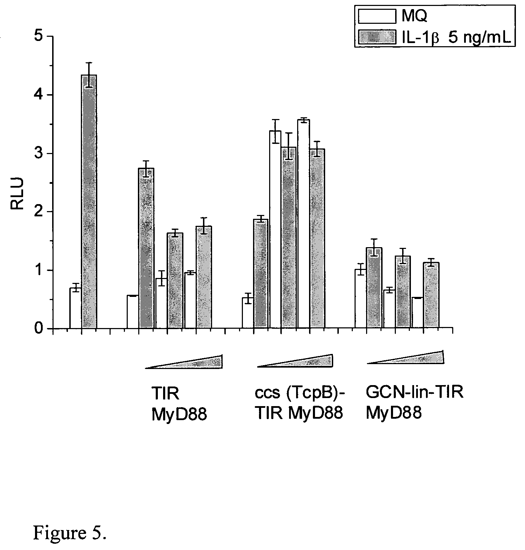

Figure 5. For determining the inhibitory effect of the fusion polypeptides comprising TIR and dimerization domain in comparison with TIR domain on ligand induced IL-1R signaling: 10 ng Rluc, 50 ng NF-κB -Fluc; 5 ng, 75 ng, 145 ng of the investigated plasmid
Figure 6. For determining the effect of the fusion polypeptides comprising TIR and dimerization domain in comparison with TIR domain on ligand-induced TNFα-R signaling: 10 ng Rluc, 50 ng NF-κB -Fluc; 5 ng, 35 ng, 75 ng of the investigated plasmid
Figure 7. For determining the effect of the added segment for membrane localization myr-TAT to GCN-lin-TIR MyD88:
   a) on its constitutive activity: 10 ng Rluc; 50 ng NF-κB -Fluc; 5 ng of the investigated plasmid
   b) on inhibitory potential on ligand-induced TLR4/MD2 signaling: 1 ng TLR4; 1 ng MD2; 10 ng Rluc; 50 ng NF-κB -Fluc; 1 ng, 10 ng, 20 ng of the investigated plasmid

The medium was changed 6 hours after transfection except in cases where the effect of fusion polypeptides comprising TIR and dimerization domain on TLR9, IL-1R and TNFα-R was observed. In these cases, the medium was changed 24 hours after transfection.

After the medium was changed, the cells were stimulated with appropriate TLR agonists as indicated in Figures 1-6. The cells were left stimulated for 16 hours except in cases where the effect of fusion polypeptides comprising TIR and dimerization domain on TLR9, IL-1R and TNFα-R was observed. In these cases, the cells were stimulated for 24 hours.

In the experiments determining the constitutive activity of the fusion polypeptides of the present invention, the medium was changed 6 hours after transfection and the cells were further incubated for 16 hours.

Cells were then lysed with a buffer according to the manufacturer's instructions (Promega). First, activity of firefly luciferase (Fluc) was measured, and then the activity of Renilla luciferase (Rluc; phRL-TK http://www.promega.com/vectors/prltk.txf). The dual luciferase method used is described in the manufacturer's instructions (Promega). Rluc activity indicates the proportion of successfully transfected cells, while Fluc activity shows activation of NF-κB or IFNβ transcription factors. The ratio of Fluc/Rluc (RLU - relative luciferase units) is a normalized measure of the number of activated cells in relation to the transfected cells.

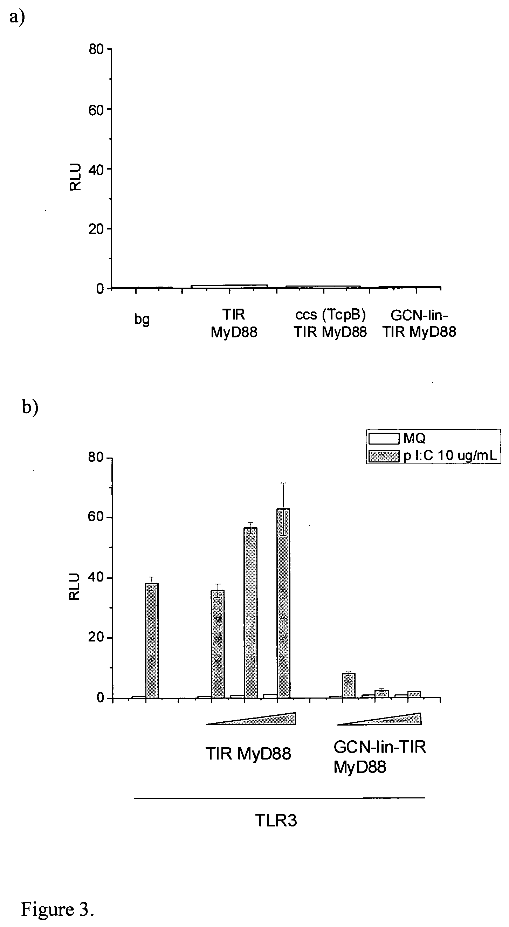

The results shown in Figure 2 demonstrate that the constructs containing TIR domain with added dimerization domain wherein the two are connected by linker peptide exhibit far greater inhibitory effect than monomeric TIR domains confirming the inventors idea that the constructs of TIR domains with dimerization domain wherein the two are connected by a linker peptide inhibit TLR signalization more efficiently than monomeric TIR domains. Furthermore, as shown in Figures 2 and 3 the construct GCN-lin-TIR MyD88 is also capable of inhibiting intracellular TLR3 and TLR9 receptors, indicating its potential for treatment of diseases involving improper/excessive endogenous TLR activation, e.g. TLR9 is involved in SLE. The importance of linker peptide inserted between dimerization segment and TIR domain is shown in Figure 4. The fusion polypeptides comprising TIR and dimerization domain are specific for the TLR as well as IL-1R signaling (Figure 5) confirming their potential use for the treatment of inflammatory diseases involving not only TLR but also IL-1R signaling, e.g. gouty inflammation. The specificity of the fusion polypeptides comprising TIR and dimerization domain is also shown in Figure 6 indicating that they do not exhibit any effect on TNFα-R signaling. The effect of the optionally added segment enabling plasma membrane localization to the fusion polypeptides of the present invention is shown in Figure 7.

### Example 2: Recombinant production of the fusion polypeptide comprising TIR and dimerization domain containing protein transduction domain for the delivery into target cells

In one embodiment of the present invention, also the fusion polypeptide comprising TIR and dimerization segment which is produced as recombinant protein, isolated and purified can be used for the delivery to the target cells where it is acts as an inhibitor of the TLR/IL-1R signaling pathway. In this embodiment, the fusion polypeptide comprising TIR and dimerization domain additionally containing protein transduction domain for the rapid and efficient transduction into target cells is preferably used. To demonstrate, we have produced the recombinant polypeptide TAT-GCN-lin-TIR MyD88 (SEQ ID NO: 10). Plasmid encoding open reading frame of TAT-GCN-lin-TIR MyD88 was transformed with chemical transformation into competent *E. coli* BL21 (DE3) pLysS cells. Selected bacterial colony grown on LB plate with selected antibiotic (ampicillin) was inoculated into 10 mL of LB broth supplemented with selected antibiotic. After several hours of growth at 37°C 10-100 µL of the culture was inoculated into 100 mL of selected growth media and left overnight shaking at 37°C. Next day the overnight culture was diluted 20-50-times reaching the OD₆₀₀ of diluted culture between 0.1 and 0.2. Culture flask with 500 ml of diluted culture was put on the shaker and bacteria were grown until OD₆₀₀ reached 0.6-0.8 when protein expression was induced by addition of inducer IPTG (1 mM). Four hours after induction culture broth was centrifuged and bacterial cells were resuspended in lysis buffer (Tris pH 8.0 0.1% deoxycholate supplemented with protease inhibitor coctail) and frozen at -80°C for at least overnight. Thawed cell suspension was further lysed with sonication and then centrifuged. Insoluble fraction and supernatant were checked for expression of TAT-GCN-lin-TIR MyD88 by SDS-PAGE and Western blot using anti-His-tag antibodies as primary antibodies. TAT-GCN-lin-TIR MyD88 was isolated from insoluble cell fraction. The insoluble fraction was washed twice and then dissolved in 6 M GdnHCl, 10 mM β-mercaptoethanol, 10 mM TRIS, 100 mM sodium phosphate, pH 8. The supernatant which remained after the centrifugation was poured onto C5 reverse phase column from which the TAT-GCN-lin-TIR MyD88 was isolated. The isolated protein was dialysed against MQ and concentrated. The purity of the protein was checked by SDS polyacrilamide gel electrophoresis (Figure 8 a). The purified protein can be efficiently transduced into the target cells where it acts as an inhibitor of the TLR signaling pathway.

### Example 3: The effect of the recombinant fusion polypeptide comprising TIR and dimerization domain additionally containing protein transduction domain on the TLR5 signalization on HEK293 cells

In order to determine the effect of the recombinant fusion polypeptide comprising TIR and dimerization domain TAT-GCN-lin-TIR MyD88 on the TLR signaling, HEK293 cells were plated on the 96 well plate and the next day transfected with 20 ng TLR5, 10 ng Rluc and 50 ng Fluc. 24 hours after the transfection the medium was changed and the recombinant TAT-GCN-lin-TIR MyD88 was added in final concentration 50 µM. After 3 hours of incubation, the cells were stimulated with flagellin in final concentration 10 ng/mL and further incubated for 16 hours. Cells were then lysed and the luminescence was measured. The detailed description of dual luciferase assay is described above. The results shown in Figure 8 b) demonstrate the inhibitory effect of recombinant TAT-GCN-lin-TIR MyD88 which acts inhibitory on the TLR5 signaling pathway on HEK293 cells. TAT-GCN-lin-TIR MyD88 could therefore be used for manufacturing a drug used for the treatment of inflammatory conditions resulting from improper and/or excessive TLR signaling employing delivery via protein transduction.

## Claims

1. Fusion polypeptide comprising a TIR domain and a dimerization domain, wherein the TIR domain and the dimerization domain are connected by a linker peptide, and wherein the dimerization domain has the sequence of SEQ ID NO: 16.

2. The fusion polypeptide according to claim 1, wherein the TIR domain is selected from the group consisting of: homologous animal, human or viral Toll/Interleukin I domains of TLR signaling adapters, Toll-like receptors, Interleukin-1 receptor family members, TIR domain containing viral proteins.

3. The fusion polypeptide according to claim 1 or 2, wherein the TIR domain is selected from the TIR domains of MyD88, TRIF, TRAM, TIRAP and SARM.

4. The fusion polypeptide according to claim 3, wherein the TIR domain is the TIR domain of MyD88, preferably having the sequence of SEQ ID NO: 12.

5. The fusion polypeptide according to any one of claims 1 to 4, wherein the peptide linker is from 3 to 40 amino acids long, preferably from 15 to 30 amino acids, more preferably it is 25 amino acids long, and wherein the amino acids are preferably selected from the group consisting of: serine, glycine, threonine, proline, valine, alanine, glutamine, asparagine, glutamate, aspartate residues.

6. The fusion polypeptide according to any one of claims 1 to 5, wherein the dimerization domain is placed at the N-terminus of the TIR domain.

7. The fusion polypeptide according to any one of claims 1 to 6, wherein the fusion polypeptide further contains a segment enabling plasma membrane localization.

8. The fusion polypeptide according to any one of claims 1 to 7, wherein the fusion polypeptide further contains a protein transduction domain, selected from the group consisting of: cationic peptide sequences, such as PTD of HIV-1 TAT protein, the herpes simplex virus 1 (HSV-1) DNA-binding protein VP22 and the Drosophila Antennapedia (Antp) homeotic transcription factor; preferably PTD of HIV-1 TAT protein, preferably having the sequence of SEQ ID NO: 22.

9. Nucleic acid encoding the fusion polypeptide according to any one of claims 1 to 8, wherein the nucleic acid is operatively linked to the regulatory elements, promoter and terminator to promote expression of the fusion polypeptide in a host organism.

10. Host cell comprising the nucleic acid according to claim 9 expressing the fusion polypeptide according to any one of claims 1 to 8.

11. The fusion polypeptide according to any one of claims 1 to 8, wherein said fusion polypeptide is produced in, isolated and purified from the host cell according to claim 10.

12. The fusion polypeptide according to any one of claims 1 to 8 or 11 for use in the treatment of inflammatory conditions, such as bacterial, viral, fungal protozoal infectious diseases, sepsis and septic shock, autoimmune diseases such as systemic lupus erythematosis, rheumatoid arthritis, Crohn's disease, inflammatory bowel disease, ulcerative colitis, insulin resistance, cancer, neuropathic pain, neuroinflammation, alcohol induced inflammation, ischaemia, hypoxia, haemorrhage, atherosclerosis, asthma, allergy, graft rejection, akylous spondilytis, resulting from improper and/or excessive TLR signaling or treatment of gouty inflammation involving ILI-R/MyD88 signaling.

13. The nucleic acid according to claim 9 encoding a fusion polypeptide according to any one of claims 1 to 6 for use in the treatment of inflammatory conditions, such as bacterial, viral, fungal protozoal infectious diseases, sepsis and septic shock, autoimmune diseases such as systemic lupus erythematosis, rheumatoid arthritis, Crohn's disease, inflammatory bowel disease, ulcerative colitis, insulin resistance, cancer, neuropathic pain, neuroinflammation, alcohol induced inflammation, ischaemia, hypoxia, haemorrhage, atherosclerosis, asthma, allergy, graft rejection, akylous spondilytis, resulting from improper and/or excessive TLR signaling resulting from improper and/or excessive TLR signaling treatment or treatment of gouty inflammation involving ILI-R/MyD88 signaling employing delivery to the target cells including but not limited to lipofection, microinjection, biolistics, immunolipofection, electroporation, gene bombardment, viral delivery.

14. The fusion polypeptide according to any one of claims 1 to 8 or 11 or the nucleic acid according to claim 7 for use in a method of inhibiting TLR/IL-IR cellular signaling in human or animal cells.

15. An *in-vitro* method for inhibitiing TLR/IL-IR cellular signaling in human or animal cells using the nucleic acid according to claim 9.

## Patentansprüche

1. Fusionspolypeptid, umfassend eine TIR-Domäne und eine Dimerisierungsdomäne, wobei die TIR-Domäne und die Dimerisierungsdomäne durch ein Linker-Peptid verbunden sind und wobei die Dimerisierungsdomäne die Sequenz der SED ID NO: 16 aufweist.

2. Fusionspolypeptid gemäß Anspruch 1, wobei die TIR-Domäne aus der Gruppe ausgewählt ist, bestehend aus: homologen tierischen, menschlichen oder viralen Toll/Interleukin-I-Domänen von TLR-Signaltransduktionsadaptoren, Toll-like-Rezeptoren, Mitgliedern der Interleukin-I-Rezeptorfamilie, viralen Proteinen, die TIR-Domänen enthalten.

3. Fusionspolypeptid gemäß Anspruch 1 oder 2, wobei die TIR-Domäne aus den TIR-Domänen von MyD88, TRIF, TRAM, TIRAP und SARM ausgewählt ist.

4. Fusionspolypeptid gemäß Anspruch 3, wobei es sich bei der TIR-Domäne um die TIR-Domäne von MyD88 handelt, vorzugsweise mit der Sequenz der SEQ ID NO: 12.

5. Fusionspolypeptid gemäß einem der Ansprüche 1 bis 4, wobei der Peptid-Linker 3 bis 40 Aminosäuren, vorzugsweise 15 bis 30 Aminosäuren lang ist, stärker bevorzugt ist er 25 Aminosäuren lang, und wobei die Aminosäuren vorzugsweise aus der Gruppe ausgewählt sind, bestehend aus: Serin-, Glycin-, Threonin-, Prolin-, Valin-, Alanin-, Glutamin-, Asparagin-, Glutamat-, Aspartatresten.

6. Fusionspolypeptid gemäß einem der Ansprüche 1 bis 5, wobei die Dimerisierungsdomäne an den N-Terminus der TIR-Domäne gefügt ist.

7. Fusionspolypeptid gemäß einem der Ansprüche 1 bis 6, wobei das Fusionspolypeptid ferner einen Abschnitt enthält, der eine Plasmamembran-Lokalisierung ermöglicht.

8. Fusionspolypeptid gemäß einem der Ansprüche 1 bis 7, wobei das Fusionspolypeptid ferner eine Proteintransduktionsdomäne enthält, ausgewählt aus der Gruppe, bestehend aus: kationischen Peptidsequenzen, wie zum Beispiel PTD des TAT-Proteins von HIV-1, dem DNA-bindenden Protein VP22 des Herpes simplex Virus 1 (HSV-1) und dem homöotischen Transkriptionsfaktor Antennapedia (Antp) von Drosophila; vorzugsweise PTD des TAT-Proteins von HIV-1, vorzugsweise mit der Sequenz der SEQ ID NO: 22.

9. Nucleinsäure, die das Fusionspolypeptid gemäß einem der Ansprüche 1 bis 8 kodiert, wobei die Nucleinsäure mit den regulatorischen Elementen, dem Promotor und Terminator funktionell verknüpft ist, um die Expression des Fusionspolypeptids in einem Wirtsorganismus zu fördern.

10. Wirtszelle, umfassend die Nucleinsäure gemäß Anspruch 9, die das Fusionspolypeptid gemäß einem der Ansprüche 1 bis 8 exprimiert.

11. Fusionspolypeptid gemäß einem der Ansprüche 1 bis 8, wobei das Fusionspolypeptid in der Wirtszelle gemäß Anspruch 10 produziert, aus ihr isoliert und gereinigt ist.

12. Fusionspolypeptid gemäß einem der Ansprüche 1 bis 8 oder 11 zur Verwendung bei der Behandlung von entzündlichen Leiden, wie zum Beispiel durch Bakterien, Viren, Pilze bzw. Protozoen ausgelöste Infektionskrankheiten, Sepsis und septischem Schock, Autoimmunerkrankungen, wie zum Beispiel systemischem Lupus erythematodes, rheumatoider Arthritis, Morbus Crohn, entzündlicher Darmerkrankung, Colitis ulcerosa, Insulinresistenz, Krebs, neuropathischen Schmerz, Nervenentzündung, alkoholinduzierter Entzündung, Ischämie, Hypoxie, Hämorrhagie, Atherosklerose, Asthma, Allergie, Transplantat-Abstoßung, Spondylitis ankylosans, verursacht durch unrichtige und/oder übermäßige TLR-Signaltransduktion, oder Behandlung von Gichtentzündung, an der ILI-R/MyD88-Signaltransduktion beteiligt ist.

13. Nucleinsäure gemäß Anspruch 9, die ein Fusionspolypeptid gemäß einem der Ansprüche 1 bis 6 kodiert, zur Verwendung bei der Behandlung von entzündlichen Leiden, wie zum Beispiel durch Bakterien, Viren, Pilze bzw. Protozoen ausgelöste Infektionskrankheiten, Sepsis und septischem Schock, Autoimmunerkrankungen, wie zum Beispiel systemischem Lupus erythematodes, rheumatoider Arthritis, Morbus Crohn, entzündlicher Darmerkrankung, Colitis ulcerosa, Insulinresistenz, Krebs, neuropathischen Schmerz, Nervenentzündung, alkoholinduzierter Entzündung, Ischämie, Hypoxie, Hämorrhagie, Atherosklerose, Asthma, Allergie, Transplantat-Abstoßung, Spondylitis ankylosans, verursacht durch unrichtige und/oder übermäßige TLR-Signaltransduktion, verursacht durch unrichtige und/oder übermäßige TLR-Signaltransduktion, oder Behandlung von Gichtentzündung, an der ILI-R/MyD88-Signaltransduktion beteiligt ist, unter Einsatz der Abgabe an die Zielzellen, einschließlich, aber nicht beschränkt auf, Lipofektion, Mikroinjektion, Biolistik, Immunlipofektion, Elektroporation, Genbeschuss, viraler Abgabe.

14. Fusionspolypeptid gemäß einem der Ansprüche 1 bis 8 oder 11 oder Nucleinsäure gemäß Anspruch 9 zur Verwendung bei einem Verfahren zum Hemmen der zellulären TLR/IL-IR-Signaltransduktion in menschlichen oder tierischen Zellen.

15. *In-vitro*-Verfahren zum Hemmen der zellulären TLR/IL-IR Signaltransduktion in menschlichen oder tierischen Zellen unter Verwendung der Nucleinsäure gemäß Anspruch 9.

## Revendications

1. Polypeptide de fusion comprenant un domaine de récepteur de type Toll-interleukine-1 TIR, soit Toll-Interleukin-1 Receptor, et un domaine de dimérisation, dans lequel le domaine TIR et le domaine de dimérisation sont connectés par un segment de liaison peptidique, et dans lequel le domaine de dimérisation présente une séquence SEQ ID n°16.

2. Polypeptide de fusion selon la revendication 1, dans lequel le domaine TIR est sélectionné parmi l'ensemble constitué par : les domaines Toll/interleukine I d'animaux homologues, humains ou viraux d'adaptateurs de signalisation de récepteurs de type Toll TLR, soit Toll-Like Receptor, les récepteurs TLR, les membres de la famille des récepteurs d'interleukine-1, et les protéines virales contenant un domaine TIR.

3. Polypeptide de fusion selon la revendication 1 ou 2, dans lequel le domaine TIR est sélectionné parmi les domaines TIR de type MyD88, TRIF, TRAM, TIRAP et SARM.

4. Polypeptide de fusion selon la revendication 3, dans lequel le domaine TIR est le domaine TIR de type MyD88, répondant de préférence à la séquence SEQ ID n°12.

5. Polypeptide de fusion selon l'une quelconque des revendications 1 à 4, dans lequel le segment de liaison peptidique présente une longueur de 3 à 40 acides aminés, de préférence de 15 à 30 acides aminés, et de préférence encore de 25 acides aminés, et dans lequel les acides aminés sont de préférence sélectionnés parmi l'ensemble constitué par des résidus : sérine, glycine, thréonine, proline, valine, alanine, glutamine, asparagine, glutamate et aspartate.

6. Polypeptide de fusion selon l'une quelconque des revendications 1 à 5, dans lequel le domaine de dimérisation est placé à la terminaison N du domaine TIR.

7. Polypeptide de fusion selon l'une quelconque des revendications 1 à 6, dans lequel le polypeptide de fusion contient en outre un segment qui habilite la localisation de la membrane plasmique.

8. Polypeptide de fusion selon l'une quelconque des revendications 1 à 7, dans lequel le polypeptide de fusion contient en outre un domaine de transduction protéique PTD, soit Protein Transduction Domain, sélectionné parmi l'ensemble constitué par : les séquences peptidiques cationiques, telles que le PTD de la protéine HIV-1 TAT, la protéine VP22 de liaison ADN du virus de l'herpès simplex 1 (HSV-1), et le facteur de transcription homéotique Antennapedia (Antp) de la drosophile ; de préférence le PTD de la protéine HIV-1 TAT, comportant de préférence la séquence SEQ ID n°22.

9. Acide nucléique codant le polypeptide de fusion selon l'une quelconque des revendications 1 à 8, dans lequel l'acide nucléique est lié fonctionnellement aux éléments régulateurs, au promoteur et au terminateur pour promouvoir l'expression du polypeptide de fusion dans un organisme hôte.

10. Cellule hôte comprenant l'acide nucléique selon la revendication 9 qui exprime le polypeptide de fusion selon l'une quelconque des revendications 1 à 8.

11. Polypeptide de fusion selon l'une quelconque des revendications 1 à 8, dans lequel ledit polypeptide de fusion est produit, isolé et purifié à partir de la cellule hôte selon la revendication 10.

12. Polypeptide de fusion selon l'une quelconque des revendications 1 à 8 ou 11, destiné à être utilisé dans le traitement de conditions inflammatoires telles que les maladies infectieuses bactériennes, virales, fongiques ou protozoaires, un sepsis et un choc septique, les maladies auto-immunes telles que le lupus érythémateux disséminé, la polyarthrite rhumatoïde, la maladie de Crohn, la maladie inflammatoire intestinale chronique, la rectocolite hémorragique, la résistance à l'insuline, le cancer, les douleurs neuropathiques, les neuro-inflammations, les inflammations induites par l'alcool, l'ischémie, l'hypoxie, l'hémorragie, l'athérosclérose, l'asthme, l'allergie, le rejet de greffe, la spondylarthrite ankylosante, résultant d'une signalisation TLR incorrecte et/ou excessive ou d'un traitement d'inflammation liée à la goutte impliquant une signalisation ILI-R/MyD88.

13. Acide nucléique selon la revendication 9 codant un polypeptide de fusion selon l'une quelconque des revendications 1 à 6 destiné à être utilisé dans le traitement de conditions inflammatoires telles que les maladies infectieuses bactériennes, virales, fongiques ou protozoaires, un sepsis et un choc septique, les maladies auto-immunes telles que le lupus érythémateux disséminé, la polyarthrite rhumatoïde, la maladie de Crohn, la maladie inflammatoire intestinale chronique, la rectocolite hémorragique, la résistance à l'insuline, le cancer, les douleurs neuropathiques, les neuro-inflammations, les inflammations induites par l'alcool, l'ischémie, l'hypoxie, l'hémorragie, l'athérosclérose, l'asthme, l'allergie, le rejet de greffe, la spondylarthrite ankylosante, résultant d'une signalisation TLR incorrecte et/ou excessive ou d'un traitement d'inflammation liée à la goutte impliquant une signalisation ILI-R/MyD88 qui emploie une administration aux cellules cibles comprenant, sans y être limitée, une lipofection, une micro-injection, un transfert biolistique, une immunolipofection, une électroporation, un bombardement génétique et une administration virale.

14. Polypeptide de fusion selon l'une quelconque des revendications 1 à 8 ou 11 ou acide nucléique selon la revendication 7, destiné à une utilisation dans un procédé d'inhibition de la signalisation cellulaire TLR/IL-IR dans des cellules humaines ou animales.

15. Procédé *in-vitro* pour inhiber la signalisation cellulaire TLR/IL-IR dans des cellules humaines ou animales en utilisant l'acide nucléique selon la revendication 9.


### SEQUENCE LISTING

<110> National Institute of Chemistry, Ljubljana, Slovenia
   Jerala, Roman
   Fekonja, Ota
<120> Fusion polypeptides comprising TIR and dimerization domain for modulation of TLR/innate immunity signaling
<130> -
<160> 22
<170> PatentIn version 3.5
<210> 1
   <211> 801
   <212> DNA
   <213> artificial
<220>
   <223> ccs (TcpB)-TIR MyD88
<220>
   <221> CDS
   <222> (1)..(801)
<400> 1
   atg gac tac aaa gac gat gac gac aag atg tct aaa gag aaa caa gcc          48
   Met Asp Tyr Lys Asp Asp Asp Asp Lys Met Ser Lys Glu Lys Gln Ala
   1               5                   10                  15
   caa tca aaa gcc cac aaa gcc caa caa gct atc agt tca gca aaa tca          96
   Gln Ser Lys Ala His Lys Ala Gln Gln Ala Ile Ser Ser Ala Lys Ser
               20                  25                  30
   ctc tcc aca cag aaa agc aaa atg tca gag ctt gag cgc gcc acg agg         144
   Leu Ser Thr Gln Lys Ser Lys Met Ser Glu Leu Glu Arg Ala Thr Arg
           35                  40                  45
   gat ggt gcc gcg att ggc aag aag cga gcg gat atc gcc aaa aaa att         192
   Asp Gly Ala Ala Ile Gly Lys Lys Arg Ala Asp Ile Ala Lys Lys Ile
       50                  55                  60
   gcc gat aag gca aaa cag tta agc tcc tat cag gct aag caa ttc aaa         240
   Ala Asp Lys Ala Lys Gln Leu Ser Ser Tyr Gln Ala Lys Gln Phe Lys
   65                  70                  75                  80
   gct gat gag cag gct gtt aaa aag gtc gcg cag gag caa aag cgt tta         288
   Ala Asp Glu Gln Ala Val Lys Lys Val Ala Gln Glu Gln Lys Arg Leu
                   85                  90                  95
   tca gat gag cga acc aag cat gaa gct ttc atc aaa caa tca ttg agc         336
   Ser Asp Glu Arg Thr Lys His Glu Ala Phe Ile Lys Gln Ser Leu Ser
               100                 105                 110
   tcc atg cga aca act gcg agc gcg act atg gaa gca gaa gaa gaa gct         384
   Ser Met Arg Thr Thr Ala Ser Ala Thr Met Glu Ala Glu Glu Glu Ala
       115                 120                 125
   agc gca cgt ttc gat gcc ttc atc tgc tat tgc ccc agc gac atc cag        432
   Ser Ala Arg Phe Asp Ala Phe Ile Cys Tyr Cys Pro Ser Asp Ile Gln
       130                 135                 140
   ttt gtg cag gag atg atc cgg caa ctg gaa cag aca aac tat cga ctg        480
   Phe Val Gln Glu Met Ile Arg Gln Leu Glu Gln Thr Asn Tyr Arg Leu
   145                 150                 155                 160
   aag ttg tgt gtg tct gac cgc gat gtc ctg cct ggc acc tgt gtc tgg        528
   Lys Leu Cys Val Ser Asp Arg Asp Val Leu Pro Gly Thr Cys Val Trp
                   165                 170                 175
   tct att gct agt gag ctc atc gaa aag agg tgc cgc cgg atg gtg gtg        576
   Ser Ile Ala Ser Glu Leu Ile Glu Lys Arg Cys Arg Arg Met Val Val
               180                 185                 190
   gtt gtc tct gat gat tac ctg cag agc aag gaa tgt gac ttc cag acc        624
   Val Val Ser Asp Asp Tyr Leu Gln Ser Lys Glu Cys Asp Phe Gln Thr
           195                 200                 205
   aaa ttt gca ctc agc ctc tct cca ggt gcc cat cag aag cga ctg atc        672
   Lys Phe Ala Leu Ser Leu Ser Pro Gly Ala His Gln Lys Arg Leu Ile
       210                 215                 220
   ccc atc aag tac aag gca atg aag aaa gag ttc ccc agc atc ctg agg        720
   Pro Ile Lys Tyr Lys Ala Met Lys Lys Glu Phe Pro Ser Ile Leu Arg
   225                 230                 235                 240
   ttc atc act gtc tgc gac tac acc aac ccc tgc acc aaa tct tgg ttc        768
   Phe Ile Thr Val Cys Asp Tyr Thr Asn Pro Cys Thr Lys Ser Trp Phe
                   245                 250                 255
   tgg act cgc ctt gcc aag gcc ttg tcc ctg ccc                            801
   Trp Thr Arg Leu Ala Lys Ala Leu Ser Leu Pro
               260                 265
<210> 2
   <211> 267
   <212> PRT
   <213> artificial
<220>
   <223> Synthetic Construct
<400> 2
   Met Asp Tyr Lys Asp Asp Asp Asp Lys Met Ser Lys Glu Lys Gln Ala
   1               5                   10                  15
   Gln Ser Lys Ala His Lys Ala Gln Gln Ala Ile Ser Ser Ala Lys Ser
               20                  25                  30
   Leu Ser Thr Gln Lys Ser Lys Met Ser Glu Leu Glu Arg Ala Thr Arg
           35                  40                  45
   Asp Gly Ala Ala Ile Gly Lys Lys Arg Ala Asp Ile Ala Lys Lys Ile
       50                  55                  60
   Ala Asp Lys Ala Lys Gln Leu Ser Ser Tyr Gln Ala Lys Gln Phe Lys
   65                  70                  75                  80
   Ala Asp Glu Gln Ala Val Lys Lys Val Ala Gln Glu Gln Lys Arg Leu
                   85                  90                  95
   Ser Asp Glu Arg Thr Lys His Glu Ala Phe Ile Lys Gln Ser Leu Ser
               100                 105                 110
   Ser Met Arg Thr Thr Ala Ser Ala Thr Met Glu Ala Glu Glu Glu Ala
           115                 120                 125
   Ser Ala Arg Phe Asp Ala Phe Ile Cys Tyr Cys Pro Ser Asp Ile Gln
       130                 135                 140
   Phe Val Gln Glu Met Ile Arg Gln Leu Glu Gln Thr Asn Tyr Arg Leu
   145                 150                 155                 160
   Lys Leu Cys Val Ser Asp Arg Asp Val Leu Pro Gly Thr Cys Val Trp
                   165                 170                 175
   Ser Ile Ala Ser Glu Leu Ile Glu Lys Arg Cys Arg Arg Met Val Val
               180                 185                 190
   Val Val Ser Asp Asp Tyr Leu Gln Ser Lys Glu Cys Asp Phe Gln Thr
           195                 200                 205
   Lys Phe Ala Leu Ser Leu Ser Pro Gly Ala His Gln Lys Arg Leu Ile
       210                 215                 220
   Pro Ile Lys Tyr Lys Ala Met Lys Lys Glu Phe Pro Ser Ile Leu Arg
   225                 230                 235                 240
   Phe Ile Thr Val Cys Asp Tyr Thr Asn Pro Cys Thr Lys Ser Trp Phe
                   245                 250                 255
   Trp Thr Arg Leu Ala Lys Ala Leu Ser Leu Pro
               260                 265
<210> 3
   <211> 516
   <212> DNA
   <213> artificial
<220>
   <223> GCN-TIR MyD88
<220>
   <221> CDS
   <222> (1)..(516)
<400> 3
   atg agg atg aag cag ctg gaa gat aag gtg gag gaa ctg ctg tcc aag        48
   Met Arg Met Lys Gln Leu Glu Asp Lys Val Glu Glu Leu Leu Ser Lys
   1               5                   10                  15
   aac tac cac ctg gaa aac gag gtg gcc cgg ctg aag aaa ctg gtc ggc        96
   Asn Tyr His Leu Glu Asn Glu Val Ala Arg Leu Lys Lys Leu Val Gly
               20                  25                  30
   gag aga gca cgt ttc gat gcc ttc atc tgc tat tgc ccc agc gac atc       144
   Glu Arg Ala Arg Phe Asp Ala Phe Ile Cys Tyr Cys Pro Ser Asp Ile
           35                  40                  45
   cag ttt gtg cag gag atg atc cgg caa ctg gaa cag aca aac tat cga       192
   Gln Phe Val Gln Glu Met Ile Arg Gln Leu Glu Gln Thr Asn Tyr Arg
       50                  55                  60
   ctg aag ttg tgt gtg tct gac cgc gat gtc ctg cct ggc acc tgt gtc       240
   Leu Lys Leu Cys Val Ser Asp Arg Asp Val Leu Pro Gly Thr Cys Val
   65                  70                  75                  80
   tgg tct att gct agt gag ctc atc gaa aag agg tgc cgc cgg atg gtg       288
   Trp Ser Ile Ala Ser Glu Leu Ile Glu Lys Arg Cys Arg Arg Met Val
                   85                  90                  95
   gtg gtt gtc tct gat gat tac ctg cag agc aag gaa tgt gac ttc cag       336
   Val Val Val Ser Asp Asp Tyr Leu Gln Ser Lys Glu Cys Asp Phe Gln
               100                 105                 110
   acc aaa ttt gca ctc agc ctc tct cca ggt gcc cat cag aag cga ctg       384
   Thr Lys Phe Ala Leu Ser Leu Ser Pro Gly Ala His Gln Lys Arg Leu
           115                 120                 125
   atc ccc atc aag tac aag gca atg aag aaa gag ttc ccc agc atc ctg       432
   Ile Pro Ile Lys Tyr Lys Ala Met Lys Lys Glu Phe Pro Ser Ile Leu
       130                 135                 140
   agg ttc atc act gtc tgc gac tac acc aac ccc tgc acc aaa tct tgg       480
   Arg Phe Ile Thr Val Cys Asp Tyr Thr Asn Pro Cys Thr Lys Ser Trp
   145                 150                 155                 160
   ttc tgg act cgc ctt gcc aag gcc ttg tcc ctg ccc                       516
   Phe Trp Thr Arg Leu Ala Lys Ala Leu Ser Leu Pro
                   165                 170
<210> 4
   <211> 172
   <212> PRT
   <213> artificial
<220>
   <223> Synthetic Construct
<400> 4
   Met Arg Met Lys Gln Leu Glu Asp Lys Val Glu Glu Leu Leu Ser Lys
   1               5                   10                  15
   Asn Tyr His Leu Glu Asn Glu Val Ala Arg Leu Lys Lys Leu Val Gly
               20                  25                  30
   Glu Arg Ala Arg Phe Asp Ala Phe Ile Cys Tyr Cys Pro Ser Asp Ile
           35                  40                  45
   Gln Phe Val Gln Glu Met Ile Arg Gln Leu Glu Gln Thr Asn Tyr Arg
       50                  55                  60
   Leu Lys Leu Cys Val Ser Asp Arg Asp Val Leu Pro Gly Thr Cys Val
   65                  70                  75                  80
   Trp Ser Ile Ala Ser Glu Leu Ile Glu Lys Arg Cys Arg Arg Met Val
                   85                  90                  95
   Val Val Val Ser Asp Asp Tyr Leu Gln Ser Lys Glu Cys Asp Phe Gln
               100                 105                 110
   Thr Lys Phe Ala Leu Ser Leu Ser Pro Gly Ala His Gln Lys Arg Leu
           115                 120                 125
   Ile Pro Ile Lys Tyr Lys Ala Met Lys Lys Glu Phe Pro Ser Ile Leu
       130                 135                 140
   Arg Phe Ile Thr Val Cys Asp Tyr Thr Asn Pro Cys Thr Lys Ser Trp
   145                 150                 155                 160
   Phe Trp Thr Arg Leu Ala Lys Ala Leu Ser Leu Pro
                   165                 170
<210> 5
   <211> 603
   <212> DNA
   <213> artificial
<220>
   <223> GCN-lin-TIR MyD88
<220>
   <221> CDS
   <222> (1)..(603)
<400> 5
   atg agg atg aag cag ctg gaa gat aag gtg gag gaa ctg ctg tcc aag      48
   Met Arg Met Lys Gln Leu Glu Asp Lys Val Glu Glu Leu Leu Ser Lys
   1               5                   10                  15
   aac tac cac ctg gaa aac gag gtg gcc cgg ctg aag aaa ctg gtc ggc      96
   Asn Tyr His Leu Glu Asn Glu Val Ala Arg Leu Lys Lys Leu Val Gly
               20                  25                  30
   gag aga ggt acc ggc tcc gag gga aag tcc tct ggc tcc gga tct gag      144
   Glu Arg Gly Thr Gly Ser Glu Gly Lys Ser Ser Gly Ser Gly Ser Glu
           35                  40                  45
   tcc aag gtg acc gac tct ggc tcc gag acc ggc tcc tct gga tcc gca      192
   Ser Lys Val Thr Asp Ser Gly Ser Glu Thr Gly Ser Ser Gly Ser Ala
       50                  55                  60
   cgt ttc gat gcc ttc atc tgc tat tgc ccc agc gac atc cag ttt gtg      240
   Arg Phe Asp Ala Phe Ile Cys Tyr Cys Pro Ser Asp Ile Gln Phe Val
   65                  70                  75                  80
   cag gag atg atc cgg caa ctg gaa cag aca aac tat cga ctg aag ttg      288
   Gln Glu Met Ile Arg Gln Leu Glu Gln Thr Asn Tyr Arg Leu Lys Leu
                   85                  90                  95
   tgt gtg tct gac cgc gat gtc ctg cct ggc acc tgt gtc tgg tct att      336
   Cys Val Ser Asp Arg Asp Val Leu Pro Gly Thr Cys Val Trp Ser Ile
               100                 105                 110
   gct agt gag ctc atc gaa aag agg tgc cgc cgg atg gtg gtg gtt gtc      384
   Ala Ser Glu Leu Ile Glu Lys Arg Cys Arg Arg Met Val Val Val Val
           115                 120                 125
   tct gat gat tac ctg cag agc aag gaa tgt gac ttc cag acc aaa ttt      432
   Ser Asp Asp Tyr Leu Gln Ser Lys Glu Cys Asp Phe Gln Thr Lys Phe
       130                 135                 140
   gca ctc agc ctc tct cca ggt gcc cat cag aag cga ctg atc ccc atc      480
   Ala Leu Ser Leu Ser Pro Gly Ala His Gln Lys Arg Leu Ile Pro Ile
   145                 150                 155                 160
   aag tac aag gca atg aag aaa gag ttc ccc agc atc ctg agg ttc atc      528
   Lys Tyr Lys Ala Met Lys Lys Glu Phe Pro Ser Ile Leu Arg Phe Ile
                   165                 170                 175
   act gtc tgc gac tac acc aac ccc tgc acc aaa tct tgg ttc tgg act      576
   Thr Val Cys Asp Tyr Thr Asn Pro Cys Thr Lys Ser Trp Phe Trp Thr
               180                 185                 190
   cgc ctt gcc aag gcc ttg tcc ctg ccc                                  603
   Arg Leu Ala Lys Ala Leu Ser Leu Pro
           195                 200
<210> 6
   <211> 201
   <212> PRT
   <213> artificial
<220>
   <223> Synthetic Construct
<400> 6
   Met Arg Met Lys Gln Leu Glu Asp Lys Val Glu Glu Leu Leu Ser Lys
   1               5                   10                  15
   Asn Tyr His Leu Glu Asn Glu Val Ala Arg Leu Lys Lys Leu Val Gly
               20                  25                  30
   Glu Arg Gly Thr Gly Ser Glu Gly Lys Ser Ser Gly Ser Gly Ser Glu
           35                  40                  45
   Ser Lys Val Thr Asp Ser Gly Ser Glu Thr Gly Ser Ser Gly Ser Ala
       50                  55                  60
   Arg Phe Asp Ala Phe Ile Cys Tyr Cys Pro Ser Asp Ile Gln Phe Val
   65                  70                  75                  80
   Gln Glu Met Ile Arg Gln Leu Glu Gln Thr Asn Tyr Arg Leu Lys Leu
                   85                  90                  95
   Cys Val Ser Asp Arg Asp Val Leu Pro Gly Thr Cys Val Trp Ser Ile
               100                 105                 110
   Ala Ser Glu Leu Ile Glu Lys Arg Cys Arg Arg Met Val Val Val Val
           115                 120                 125
   Ser Asp Asp Tyr Leu Gln Ser Lys Glu Cys Asp Phe Gln Thr Lys Phe
       130                 135                 140
   Ala Leu Ser Leu Ser Pro Gly Ala His Gln Lys Arg Leu Ile Pro Ile
   145                 150                 155                 160
   Lys Tyr Lys Ala Met Lys Lys Glu Phe Pro Ser Ile Leu Arg Phe Ile
                   165                 170                 175
   Thr Val Cys Asp Tyr Thr Asn Pro Cys Thr Lys Ser Trp Phe Trp Thr
               180                 185                 190
   Arg Leu Ala Lys Ala Leu Ser Leu Pro
           195                 200
<210> 7
   <211> 654
   <212> DNA
   <213> artificial
<220>
   <223> myr-TAT-GCN-lin-TIR MyD88
<220>
   <221> CDS
   <222> (1)..(654)
<400> 7
   atg ggc atc ggc aag tcc aag tat ggt cgt aaa aaa cgt cgt cag cgt         48
   Met Gly Ile Gly Lys Ser Lys Tyr Gly Arg Lys Lys Arg Arg Gln Arg
   1               5                   10                  15
   cgt cgt agg atg aag cag ctg gaa gat aag gtg gag gaa ctg ctg tcc         96
   Arg Arg Arg Met Lys Gln Leu Glu Asp Lys Val Glu Glu Leu Leu Ser
               20                  25                  30
   aag aac tac cac ctg gaa aac gag gtg gcc cgg ctg aag aaa ctg gtc        144
   Lys Asn Tyr His Leu Glu Asn Glu Val Ala Arg Leu Lys Lys Leu Val
           35                  40                  45
   ggc gag aga ggt acc ggc tcc gag gga aag tcc tct ggc tcc gga tct        192
   Gly Glu Arg Gly Thr Gly Ser Glu Gly Lys Ser Ser Gly Ser Gly Ser
       50                  55                  60
   gag tcc aag gtg acc gac tct ggc tcc gag acc ggc tcc tct gga tcc        240
   Glu Ser Lys Val Thr Asp Ser Gly Ser Glu Thr Gly Ser Ser Gly Ser
   65                  70                  75                  80
   gca cgt ttc gat gcc ttc atc tgc tat tgc ccc agc gac atc cag ttt        288
   Ala Arg Phe Asp Ala Phe Ile Cys Tyr Cys Pro Ser Asp Ile Gln Phe
                   85                  90                  95
   gtg cag gag atg atc cgg caa ctg gaa cag aca aac tat cga ctg aag        336
   Val Gln Glu Met Ile Arg Gln Leu Glu Gln Thr Asn Tyr Arg Leu Lys
               100                 105                 110
   ttg tgt gtg tct gac cgc gat gtc ctg cct ggc acc tgt gtc tgg tct        384
   Leu Cys Val Ser Asp Arg Asp Val Leu Pro Gly Thr Cys Val Trp Ser
           115                 120                 125
   att gct agt gag ctc atc gaa aag agg tgc cgc cgg atg gtg gtg gtt        432
   Ile Ala Ser Glu Leu Ile Glu Lys Arg Cys Arg Arg Met Val Val Val
       130                 135                 140
   gtc tct gat gat tac ctg cag agc aag gaa tgt gac ttc cag acc aaa        480
   Val Ser Asp Asp Tyr Leu Gln Ser Lys Glu Cys Asp Phe Gln Thr Lys
   145                 150                 155                 160
   ttt gca ctc agc ctc tct cca ggt gcc cat cag aag cga ctg atc ccc        528
   Phe Ala Leu Ser Leu Ser Pro Gly Ala His Gln Lys Arg Leu Ile Pro
                   165                 170                 175
   atc aag tac aag gca atg aag aaa gag ttc ccc agc atc ctg agg ttc        576
   Ile Lys Tyr Lys Ala Met Lys Lys Glu Phe Pro Ser Ile Leu Arg Phe
               180                 185                 190
   atc act gtc tgc gac tac acc aac ccc tgc acc aaa tct tgg ttc tgg        624
   Ile Thr Val Cys Asp Tyr Thr Asn Pro Cys Thr Lys Ser Trp Phe Trp
           195                 200                 205
   act cgc ctt gcc aag gcc ttg tcc ctg ccc                                654
   Thr Arg Leu Ala Lys Ala Leu Ser Leu Pro
       210                 215
<210> 8
   <211> 218
   <212> PRT
   <213> artificial
<220>
   <223> Synthetic Construct
<400> 8
   Met Gly Ile Gly Lys Ser Lys Tyr Gly Arg Lys Lys Arg Arg Gln Arg
   1               5                   10                  15
   Arg Arg Arg Met Lys Gln Leu Glu Asp Lys Val Glu Glu Leu Leu Ser
               20                  25                  30
   Lys Asn Tyr His Leu Glu Asn Glu Val Ala Arg Leu Lys Lys Leu Val
           35                  40                  45
   Gly Glu Arg Gly Thr Gly Ser Glu Gly Lys Ser Ser Gly Ser Gly Ser
       50                  55                  60
   Glu Ser Lys Val Thr Asp Ser Gly Ser Glu Thr Gly Ser Ser Gly Ser
   65                  70                  75                  80
   Ala Arg Phe Asp Ala Phe Ile Cys Tyr Cys Pro Ser Asp Ile Gln Phe
                   85                  90                  95
   Val Gln Glu Met Ile Arg Gln Leu Glu Gln Thr Asn Tyr Arg Leu Lys
               100                 105                 110
   Leu Cys Val Ser Asp Arg Asp Val Leu Pro Gly Thr Cys Val Trp Ser
           115                 120                 125
   Ile Ala Ser Glu Leu Ile Glu Lys Arg Cys Arg Arg Met Val Val Val
       130                 135                 140
   Val Ser Asp Asp Tyr Leu Gln Ser Lys Glu Cys Asp Phe Gln Thr Lys
   145                 150                 155                 160
   Phe Ala Leu Ser Leu Ser Pro Gly Ala His Gln Lys Arg Leu Ile Pro
                   165                 170                 175
   Ile Lys Tyr Lys Ala Met Lys Lys Glu Phe Pro Ser Ile Leu Arg Phe
               180                 185                 190
   Ile Thr Val Cys Asp Tyr Thr Asn Pro Cys Thr Lys Ser Trp Phe Trp
           195                 200                 205
   Thr Arg Leu Ala Lys Ala Leu Ser Leu Pro
       210                 215
<210> 9
   <211> 666
   <212> DNA
   <213> artificial
<220>
   <223> TAT-GCN-lin-TIR MyD88
<220>
   <221> CDS
   <222> (1)..(666)
<400> 9
   atg cat cat cat cat cat cat ggt ggt tat ggt cgt aaa aaa cgt cgt         48
   Met His His His His His His Gly Gly Tyr Gly Arg Lys Lys Arg Arg
   1               5                   10                  15
   cag cgt cgt cgt ggt ggt agg atg aag cag ctg gaa gat aag gtg gag         96
   Gln Arg Arg Arg Gly Gly Arg Met Lys Gln Leu Glu Asp Lys Val Glu
               20                  25                  30
   gaa ctg ctg tcc aag aac tac cac ctg gaa aac gag gtg gcc cgg ctg        144
   Glu Leu Leu Ser Lys Asn Tyr His Leu Glu Asn Glu Val Ala Arg Leu
           35                  40                  45
   aag aaa ctg gtc ggc gag aga ggt acc ggc tcc gag gga aag tcc tct        192
   Lys Lys Leu Val Gly Glu Arg Gly Thr Gly Ser Glu Gly Lys Ser Ser
       50                  55                  60
   ggc tcc gga tct gag tcc aag gtg acc gac tct ggc tcc gag acc ggc        240
   Gly Ser Gly Ser Glu Ser Lys Val Thr Asp Ser Gly Ser Glu Thr Gly
   65                  70                  75                  80
   tcc tct gga tcc gca cgt ttc gat gcc ttc atc tgc tat tgc ccc agc        288
   Ser Ser Gly Ser Ala Arg Phe Asp Ala Phe Ile Cys Tyr Cys Pro Ser
                   85                  90                  95
   gac atc cag ttt gtg cag gag atg atc cgg caa ctg gaa cag aca aac        336
   Asp Ile Gln Phe Val Gln Glu Met Ile Arg Gln Leu Glu Gln Thr Asn
               100                 105                 110
   tat cga ctg aag ttg tgt gtg tct gac cgc gat gtc ctg cct ggc acc         384
   Tyr Arg Leu Lys Leu Cys Val Ser Asp Arg Asp Val Leu Pro Gly Thr
           115                 120                 125
   tgt gtc tgg tct att gct agt gag ctc atc gaa aag agg tgc cgc cgg         432
   Cys Val Trp Ser Ile Ala Ser Glu Leu Ile Glu Lys Arg Cys Arg Arg
       130                 135                 140
   atg gtg gtg gtt gtc tct gat gat tac ctg cag agc aag gaa tgt gac         480
   Met Val Val Val Val Ser Asp Asp Tyr Leu Gln Ser Lys Glu Cys Asp
   145                 150                 155                 160
   ttc cag acc aaa ttt gca ctc agc ctc tct cca ggt gcc cat cag aag         528
   Phe Gln Thr Lys Phe Ala Leu Ser Leu Ser Pro Gly Ala His Gln Lys
                   165                 170                 175
   cga ctg atc ccc atc aag tac aag gca atg aag aaa gag ttc ccc agc         576
   Arg Leu Ile Pro Ile Lys Tyr Lys Ala Met Lys Lys Glu Phe Pro Ser
               180                 185                 190
   atc ctg agg ttc atc act gtc tgc gac tac acc aac ccc tgc acc aaa         624
   Ile Leu Arg Phe Ile Thr Val Cys Asp Tyr Thr Asn Pro Cys Thr Lys
           195                 200                 205
   tct tgg ttc tgg act cgc ctt gcc aag gcc ttg tcc ctg ccc                 666
   Ser Trp Phe Trp Thr Arg Leu Ala Lys Ala Leu Ser Leu Pro
       210                 215                 220
<210> 10
   <211> 222
   <212> PRT
   <213> artificial
<220>
   <223> Synthetic Construct
<400> 10
   Met His His His His His His Gly Gly Tyr Gly Arg Lys Lys Arg Arg
   1               5                   10                  15
   Gln Arg Arg Arg Gly Gly Arg Met Lys Gln Leu Glu Asp Lys Val Glu
               20                  25                  30
   Glu Leu Leu Ser Lys Asn Tyr His Leu Glu Asn Glu Val Ala Arg Leu
           35                  40                  45
   Lys Lys Leu Val Gly Glu Arg Gly Thr Gly Ser Glu Gly Lys Ser Ser
       50                  55                  60
   Gly Ser Gly Ser Glu Ser Lys Val Thr Asp Ser Gly Ser Glu Thr Gly
   65                  70                  75                  80
   Ser Ser Gly Ser Ala Arg Phe Asp Ala Phe Ile Cys Tyr Cys Pro Ser
                   85                  90                  95
   Asp Ile Gln Phe Val Gln Glu Met Ile Arg Gln Leu Glu Gln Thr Asn
               100                 105                 110
   Tyr Arg Leu Lys Leu Cys Val Ser Asp Arg Asp Val Leu Pro Gly Thr
           115                 120                 125
   Cys Val Trp Ser Ile Ala Ser Glu Leu Ile Glu Lys Arg Cys Arg Arg
       130                 135                 140
   Met Val Val Val Val Ser Asp Asp Tyr Leu Gln Ser Lys Glu Cys Asp
   145                 150                 155                 160
   Phe Gln Thr Lys Phe Ala Leu Ser Leu Ser Pro Gly Ala His Gln Lys
                   165                 170                 175
   Arg Leu Ile Pro Ile Lys Tyr Lys Ala Met Lys Lys Glu Phe Pro Ser
               180                 185                 190
   Ile Leu Arg Phe Ile Thr Val Cys Asp Tyr Thr Asn Pro Cys Thr Lys
           195                 200                 205
   Ser Trp Phe Trp Thr Arg Leu Ala Lys Ala Leu Ser Leu Pro
       210                 215                 220
<210> 11
   <211> 417
   <212> DNA
   <213> Homo sapiens
<220>
   <221> CDS
   <222> (1)..(417)
<400> 11
   atg gca cgt ttc gat gcc ttc atc tgc tat tgc ccc agc gac atc cag          48
   Met Ala Arg Phe Asp Ala Phe Ile Cys Tyr Cys Pro Ser Asp Ile Gln
   1               5                   10                  15
   ttt gtg cag gag atg atc cgg caa ctg gaa cag aca aac tat cga ctg          96
   Phe Val Gln Glu Met Ile Arg Gln Leu Glu Gln Thr Asn Tyr Arg Leu
               20                  25                  30
   aag ttg tgt gtg tct gac cgc gat gtc ctg cct ggc acc tgt gtc tgg         144
   Lys Leu Cys Val Ser Asp Arg Asp Val Leu Pro Gly Thr Cys Val Trp
           35                  40                  45
   tct att gct agt gag ctc atc gaa aag agg tgc cgc cgg atg gtg gtg         192
   Ser Ile Ala Ser Glu Leu Ile Glu Lys Arg Cys Arg Arg Met Val Val
       50                  55                  60
   gtt gtc tct gat gat tac ctg cag agc aag gaa tgt gac ttc cag acc         240
   Val Val Ser Asp Asp Tyr Leu Gln Ser Lys Glu Cys Asp Phe Gln Thr
   65                  70                  75                  80
   aaa ttt gca ctc agc ctc tct cca ggt gcc cat cag aag cga ctg atc         288
   Lys Phe Ala Leu Ser Leu Ser Pro Gly Ala His Gln Lys Arg Leu Ile
                   85                  90                  95
   ccc atc aag tac aag gca atg aag aaa gag ttc ccc agc atc ctg agg         336
   Pro Ile Lys Tyr Lys Ala Met Lys Lys Glu Phe Pro Ser Ile Leu Arg
               100                 105                 110
   ttc atc act gtc tgc gac tac acc aac ccc tgc acc aaa tct tgg ttc         384
   Phe Ile Thr Val Cys Asp Tyr Thr Asn Pro Cys Thr Lys Ser Trp Phe
           115                 120                 125
   tgg act cgc ctt gcc aag gcc ttg tcc ctg ccc                             417
   Trp Thr Arg Leu Ala Lys Ala Leu Ser Leu Pro
       130                 135
<210> 12
   <211> 139
   <212> PRT
   <213> Homo sapiens
<400> 12
   Met Ala Arg Phe Asp Ala Phe Ile Cys Tyr Cys Pro Ser Asp Ile Gln
   1               5                   10                  15
   Phe Val Gln Glu Met Ile Arg Gln Leu Glu Gln Thr Asn Tyr Arg Leu
               20                  25                  30
   Lys Leu Cys Val Ser Asp Arg Asp Val Leu Pro Gly Thr Cys Val Trp
           35                  40                  45
   Ser Ile Ala Ser Glu Leu Ile Glu Lys Arg Cys Arg Arg Met Val Val
       50                  55                  60
   Val Val Ser Asp Asp Tyr Leu Gln Ser Lys Glu Cys Asp Phe Gln Thr
   65                  70                  75                  80
   Lys Phe Ala Leu Ser Leu Ser Pro Gly Ala His Gln Lys Arg Leu Ile
                   85                  90                  95
   Pro Ile Lys Tyr Lys Ala Met Lys Lys Glu Phe Pro Ser Ile Leu Arg
               100                 105                 110
   Phe Ile Thr Val Cys Asp Tyr Thr Asn Pro Cys Thr Lys Ser Trp Phe
           115                 120                 125
   Trp Thr Arg Leu Ala Lys Ala Leu Ser Leu Pro
       130                 135
<210> 13
   <211> 354
   <212> DNA
   <213> Brucella melitensis
<220>
   <221> CDS
   <222> (1)..(354)
<400> 13
   atg tct aaa gag aaa caa gcc caa tca aaa gcc cac aaa gcc caa caa        48
   Met Ser Lys Glu Lys Gln Ala Gln Ser Lys Ala His Lys Ala Gln Gln
   1               5                   10                  15
   gct atc agt tca gca aaa tca ctc tcc aca cag aaa agc aaa atg tca        96
   Ala Ile Ser Ser Ala Lys Ser Leu Ser Thr Gln Lys Ser Lys Met Ser
               20                  25                  30
   gag ctt gag cgc gcc acg agg gat ggt gcc gcg att ggc aag aag cga       144
   Glu Leu Glu Arg Ala Thr Arg Asp Gly Ala Ala Ile Gly Lys Lys Arg
           35                  40                  45
   gcg gat atc gcc aaa aaa att gcc gat aag gca aaa cag tta agc tcc       192
   Ala Asp Ile Ala Lys Lys Ile Ala Asp Lys Ala Lys Gln Leu Ser Ser
       50                  55                  60
   tat cag gct aag caa ttc aaa gct gat gag cag gct gtt aaa aag gtc       240
   Tyr Gln Ala Lys Gln Phe Lys Ala Asp Glu Gln Ala Val Lys Lys Val
   65                  70                  75                  80
   gcg cag gag caa aag cgt tta tca gat gag cga acc aag cat gaa gct       288
   Ala Gln Glu Gln Lys Arg Leu Ser Asp Glu Arg Thr Lys His Glu Ala
                   85                  90                  95
   ttc atc aaa caa tca ttg agc tcc atg cga aca act gcg agc gcg act       336
   Phe Ile Lys Gln Ser Leu Ser Ser Met Arg Thr Thr Ala Ser Ala Thr
               100                 105                 110
   atg gaa gca gaa gaa gaa                                               354
   Met Glu Ala Glu Glu Glu
           115
<210> 14
   <211> 118
   <212> PRT
   <213> Brucella melitensis
<400> 14
   Met Ser Lys Glu Lys Gln Ala Gln Ser Lys Ala His Lys Ala Gln Gln
   1               5                   10                  15
   Ala Ile Ser Ser Ala Lys Ser Leu Ser Thr Gln Lys Ser Lys Met Ser
               20                  25                  30
   Glu Leu Glu Arg Ala Thr Arg Asp Gly Ala Ala Ile Gly Lys Lys Arg
           35                  40                  45
   Ala Asp Ile Ala Lys Lys Ile Ala Asp Lys Ala Lys Gln Leu Ser Ser
       50                  55                  60
   Tyr Gln Ala Lys Gln Phe Lys Ala Asp Glu Gln Ala Val Lys Lys Val
   65                  70                  75                  80
   Ala Gln Glu Gln Lys Arg Leu Ser Asp Glu Arg Thr Lys His Glu Ala
                   85                  90                  95
   Phe Ile Lys Gln Ser Leu Ser Ser Met Arg Thr Thr Ala Ser Ala Thr
               100                 105                 110
   Met Glu Ala Glu Glu Glu
           115
<210> 15
   <211> 99
   <212> DNA
   <213> synthetic
<220>
   <221> CDS
   <222> (1)..(99)
<400> 15
   agg atg aag cag ctg gaa gat aag gtg gag gaa ctg ctg tcc aag aac         48
   Arg Met Lys Gln Leu Glu Asp Lys Val Glu Glu Leu Leu Ser Lys Asn
   1               5                   10                  15
   tac cac ctg gaa aac gag gtg gcc cgg ctg aag aaa ctg gtc ggc gag         96
   Tyr His Leu Glu Asn Glu Val Ala Arg Leu Lys Lys Leu Val Gly Glu
               20                  25                  30
   aga                                                                     99
   Arg
<210> 16
   <211> 33
   <212> PRT
   <213> synthetic
<400> 16
   Arg Met Lys Gln Leu Glu Asp Lys Val Glu Glu Leu Leu Ser Lys Asn
   1               5                   10                  15
   Tyr His Leu Glu Asn Glu Val Ala Arg Leu Lys Lys Leu Val Gly Glu
               20                  25                  30
   Arg
<210> 17
   <211> 75
   <212> DNA
   <213> synthetic
<220>
   <221> CDS
   <222> (1)..(75)
<400> 17
   ggc tcc gag gga aag tcc tct ggc tcc gga tct gag tcc aag gtg acc         48
   Gly Ser Glu Gly Lys Ser Ser Gly Ser Gly Ser Glu Ser Lys Val Thr
   1               5                   10                  15
   gac tct ggc tcc gag acc ggc tcc tct                                     75
   Asp Ser Gly Ser Glu Thr Gly Ser Ser
               20                  25
<210> 18
   <211> 25
   <212> PRT
   <213> synthetic
<400> 18
   Gly Ser Glu Gly Lys Ser Ser Gly Ser Gly Ser Glu Ser Lys Val Thr
   1               5                   10                  15
   Asp Ser Gly Ser Glu Thr Gly Ser Ser
               20                  25
<210> 19
   <211> 21
   <212> DNA
   <213> Homo sapiens
<220>
   <221> CDS
   <222> (1)..(21)
<400> 19
   atg ggc atc ggc aag tcc aag                                           21
   Met Gly Ile Gly Lys Ser Lys
   1               5
<210> 20
   <211> 7
   <212> PRT
   <213> Homo sapiens
<400> 20
   Met Gly Ile Gly Lys Ser Lys
   1               5
<210> 21
   <211> 33
   <212> DNA
   <213> synthetic
<220>
   <221> CDS
   <222> (1)..(33)
<400> 21
   tat ggt cgt aaa aaa cgt cgt cag cgt cgt cgt                           33
   Tyr Gly Arg Lys Lys Arg Arg Gln Arg Arg Arg
   1               5                   10
<210> 22
   <211> 11
   <212> PRT
   <213> synthetic
<400> 22
   Tyr Gly Arg Lys Lys Arg Arg Gln Arg Arg Arg
   1               5                   10